# EUROPÄISCHE PATENTSCHRIFT

(11) **EP 2 314 206 B1**
(45) Veröffentlichungstag und Bekanntmachung des Hinweises auf die Patenterteilung: **05.06.2013**
(21) Anmeldenummer: 10009099.2
(22) Anmeldetag: 04.09.2006
(51) Int. Cl.: A61B 5/00, A61B 5/155

(54) **Stechsystem zur Entnahme einer Körperflüssigkeit**
Piercing system for removal of a bodily fluid
Système de piqûre pour l'extraction d'un liquide corporel

(43) Veröffentlichungstag der Anmeldung: 27.04.2011
(62) Teilanmeldung aus: 06018498.3
(73) Patentinhaber: Roche Diagnostics GmbH, 68305 Mannheim (DE); F.Hoffmann-La Roche AG, 4070 Basel (CH)
(72) Erfinder: List, Hans, 64754 Hesseneck-Kailbach (DE); Calasso, Irio, Dr., 6415 Arth (CH); Hoenes, Joachim, Dr., 64673 Zwingenberg (DE); Haar, Hans-Peter, Dr., 69168 Wiesloch (DE); Krämer, Uwe, Dr., 68549 Ilvesheim (DE); Hartig, Herbert, Dr., 67434 Neustadt (DE); Zimmer, Volker, 54497 Morbach (DE)
(74) Vertreter: Durm & Partner

(56) Entgegenhaltungen:
- EP-A1- 1 527 736
- EP-A1- 1 669 028
- EP-A2- 0 199 484
- US-A1- 2004 059 256

## Beschreibung

Die Erfindung betrifft ein Stechsystem zur Entnahme einer Körperflüssigkeit aus der Haut eines Menschen oder Tieres. Das Stechsystem besteht aus einem Nadelelement zum Einstechen in die Haut und einem Stechgerät, das einen Stechantrieb einschließt. Durch den Stechantrieb wird eine Stechbewegung eines mittels eines Kopplungsmechanismus mit dem Stechantrieb gekoppelten Nadelelementes in Einstichrichtung angetrieben.

Die mit dem Stechsystem gewonnene Körperflüssigkeit ist in der Regel Blut. In manchen Anwendungsfällen werden aber auch Proben von interstitieller Flüssigkeit gewonnen. Wenn nachfolgend Blut als Beispiel einer Körperflüssigkeit erwähnt wird, ist dies keine Beschränkung der Allgemeinheit. Blut steht nur als Beispiel für jede andere aus der Haut entnehmbare Körperflüssigkeit.

Für Diagnosezwecke werden geringe Mengen von Blut aus einem Körperteil, beispielsweise aus dem Finger oder dem Ohrläppchen, entnommen. Hierzu werden Lanzetten verwendet, die mit ihrer Spitze eine Wunde in das entsprechende Körperteil stechen. Der Einstich der Lanzette in die Haut geschieht entweder durch besonders trainiertes Personal, das den Einstich manuell vornimmt, oder durch besondere Blutentnahmesysteme, die sogenannte Stechgeräte und zugehörige Lanzetten einschließen.

Bei einfachen Stechgeräten wird die Lanzette in einer schnellen Bewegung in die Haut hinein und nach Erreichend es Umkehrpunkts aus ihr wieder herausgezogen. Anschließend wird das Stechgerät entnommen. Der Benutzer massiert dann die Haut im Bereich der Einstichstelle und drückt sie zusammen, um den Blutaustritt aus der Haut zu fördern. Dieses als "Melken" bezeichnete Zusammendrücken der Haut wird so lange fortgesetzt, bis ein genügend großer Blutstropfen aus der Haut heraustritt. In einem weiteren Schritt wird der Blutstropfen auf einen Teststreifen gebracht, um die Konzentration eines in dem Blut vorhanden Analyten, insbesondere den Glucosewert, zu bestimmen. Dieses Vorgehen ist für den Benutzer jedoch umständlich und auch unangenehm, da er die Haut in der Nähe der Wunde zusammenquetschen muss.

Insbesondere für Diabetiker, die sich mehrmals am Tag in die Haut stechen müssen, um den Glucosegehalt im Blut zu bestimmen, ist ein möglichst schmerzfreies Stechen wichtig. Ebenso wichtig ist eine vereinfachte Handhabung der Blutentnahme. Gerade das als unangenehm empfundene "Melken" sollte vermieden werden. Aus diesem Grund wurden mehrere Stechgeräte im Stand der Technik entwickelt, die eine große Austrittsöffnung für die Lanzette mit einem Durchmesser von mehreren Millimetern aufweisen. Das die Austrittsöffnung ringförmig umschließende vordere Ende des Gehäuses bildet eine Gehäuse-Hautkontaktfläche, die gegen die Haut gedrückt wird. Infolge des Drucks wölbt sich die Haut in die Öffnung des Stechgerätes hinein. Gleichzeitig wird durch den ausgeübten Anpressdruck der Innendruck im Finger (bzw. anderen Körperteil) erhöht, so dass bei einem Einstechen der Lanzette in die Haut die Körperflüssigkeit bzw. das Blut spontan austritt, ohne dass ein manuelles Massieren oder "Melken" notwendig ist. Derartige Produkte werden nachfolgend als "Stechsystem mit Expressionshilfe" bezeichnet.

Es ist bereits seit längerem bekannt, dass das Schmerzempfinden wesentlich von der Reproduzierbarkeit der Stichtiefe beeinflusst wird. Mit anderen Worten soll die Tiefe des Einstichs bei einer Mehrzahl von zeitlich getrennten Stechvorgängen (aber unveränderter Einstellung des Stechgerätes) möglichst gleich bleiben, um mit einer minimalen Stichtiefe eine ausreichende Blutmenge zu erzeugen (US-Patent 5,318,584). Die Stichtiefe wird einerseits durch die Position des Umkehrpunktes der Stechbewegung und andererseits durch die Position der Haut bei Erreichen des Umkehrpunktes bestimmt. Die Reproduzierbarkeit der Position des Umkehrpunktes hängt von der Konstruktion des Stechantriebs ab. Es sind zahlreiche Antriebskonstruktionen bekannt, die eine sehr gute Reproduzierbarkeit der Position des Umkehrpunktes gewährleisten. Hierzu gehören insbesondere Rotorantriebe, wie sie in dem US-Patent 5,318,584 und zahlreichen weiteren Dokumenten des Standes der Technik beschrieben werden. Die vorliegende Erfindung kann mit unterschiedlichen Antriebskonstruktionen kombiniert werden, sofern sie eine ausreichend gute Reproduzierbarkeit der Position des Umkehrpunktes bei mehreren Stechvorgängen und unveränderter Einstellung des Gerätes gewährleisten.

Bei gegebener Qualität des Stechantriebes hängt die Reproduzierbarkeit der Stichtiefe entscheidend davon ab, dass die Position der Haut während des Stichs reproduzierbar definiert ist. Diesbezüglich bestehen bei Stechsystem mit Expressionshilfe besondere Probleme, weil das Einwölben der Haut von der Beschaffenheit der Haut abhängig ist und deshalb von Benutzer zu Benutzer variiert. Darüber hinaus verändert die Haut ihre Eigenschaften auch bei einzelnen Benutzern beispielsweise in Abhängigkeit davon, ob sie trocken oder feucht bzw. kalt oder warm ist. Deswegen stellt die Gehäuse-Hautkontaktfläche am Innenrand der Gehäuseöffnung nur eine ungenaue Referenz für die Einstichtiefe dar.

Zur Lösung dieses Problems wird in der EP 1669028 A1 ein Referenzelement vorgeschlagen, das mittels eines Referenzelement-Kopplungsmechanismus mit dem Antrieb des Stechgerätes gekoppelt und dadurch beweglich ist. Es wird innerhalb des Stechgerätes nach vorne bewegt, bis eine an seinem vorderen Ende ausgebildete Kontaktfläche an der Haut anliegt. Dadurch wird die Variation des Einwölbens der Haut in die Gehäuseöffnung detektiert und die aus dieser Variation resultierende Einschränkung der Reproduzierbarkeit eliminiert. Gemäß der EP 1669028 soll der Kontaktdruck zwischen dem Referenzelement und der Hautoberfläche klein sein, weil ein erhöhter Druck dem Austreten von Blut aus der Einstichwunde entgegenwirkt. Außerdem wird hervorgehoben, dass das Referenzelement nach dem Einstich sehr schnell (innerhalb von höchstens 50 msec) von der Einstichwunde wegbewegt werden soll, insbesondere um eine Verschmutzung des Referenzelementes durch austretendes Blut zu vermeiden.

Gemäß einer Reihe weiterer bekannter Vorschläge sollen Funktionsverbesserungen, unter anderem hinsichtlich der Reproduzierbarkeit der Stichtiefe, dadurch erreicht werden, dass ein Stechtiefenkontrollelement gemeinsam mit einer Nadel schnell auf die Haut zubewegt wird, wobei durch den Impuls beim Auftreffen auf die Haut positive Wirkungen erreicht werden sollen.

Beispielsweise offenbart die EP 1527736 A1 ein Stechgerät, bei dem eine Stecheinheit, bestehend aus einer Lanzette und einem Stechtiefenkontrollelement, gegen die Haut bewegt wird. Während der Vortriebsphase der Lanzette wird das Stechtiefenkontrollelement mitbewegt, bis es an die Haut anschlägt. Anschließend sticht die Spitze der Lanzette in die Haut ein.

Ein vom Prinzip her ähnliches Stechgerät ist in US 6,306,152 B1 offenbart. Das Stechgerät weist eine relativ große Gehäuseöffnung auf, durch die sich die Haut in das Stechgerät hineinwölben kann. Um die Haut während des Einstichs zu spannen, wird vorgeschlagen, ein federbelastetes Kontrollelement einzusetzen, das gemeinsam mit der Lanzette während der Vortriebsphase der Stechbewegung in Richtung Haut bewegt wird. Sobald das Kontrollelement auf die Haut aufschlägt, wird die in dem Kontrollelement beweglich gelagerte Lanzette durch ihre Trägheit in Einstichrichtung weiterbewegt, bis sie an einen Anschlag des Kontrollelements anschlägt. Dabei führt sie einen Stich in die Haut aus. Die für den Vortrieb des Kontrollelements verwendete Feder zieht dann das Kontrollelement mit der Lanzette zurück, wodurch die Lanzette aus der Haut bezogen wird. Auch die US 6,929,650 B2 beschreibt ein System, bei dem die Lanzette auf dem Weg in die Haut eine Anschlagstruktur mitnimmt, die sich an der Haut abstützt und durch die die Einstichtiefe definiert wird.

Auf dieser Grundlage ist es Aufgabe der Erfindung, ein Stechsystem vorzuschlagen, bei dem das Blut nach dem Einstich ohne zusätzliche Handhabungsschritte aus der Haut austritt und der Einstich für den Patienten möglichst schmerzarm erfolgt. Ein derartiges System soll die im Stand der Technik bekannten Nachteile überwinden.

Die Aufgabe wird gelöst durch ein Stechsystem zur Entnahme einer Körperflüssigkeit aus der Haut eines Körperteils eines Menschen oder Tieres mit den Merkmalen gemäß Anspruch 1.

Das Stechsystem umfasst ein Nadelelement zum Einstechen in die Haut und ein Stechgerät, das einen Stechantrieb einschließt. Durch den Stechantrieb wird eine Stechbewegung des Nadelelementes, das mittels eines Kopplungsmechanismus mit dem Stechantrieb gekoppelt ist, in Einstichrichtung angetrieben. Das Stechgerät hat ein Gehäuse mit einer Gehäuseöffnung an seinem in Einstichrichtung vorderem Ende. Die Gehäuseöffnung wird von einer Gehäuse-Hautkontaktfläche umgeben, die bei der Benutzung des Stechgerätes gegen die Haut des Körperteils gedrückt wird.

Das Nadelelement wird in einer Vortriebsphase der Stechbewegung auf einem vorbestimmten Einstichweg in einer Einstichrichtung bewegt, bis seine Spitze in die Haut eindringt, um eine Wunde zu erzeugen. In einer Rückführungsphase der Stechbewegung nach Erreichen eines der Stichtiefe in die Haut entsprechenden Umkehrpunktes wird das Nadelelement wieder zurückgezogen. Ein vorgegebener Wert der Stichtiefe wird durch ein Stechtiefenreferenzelement mit einer Referenz-Hautkontaktfläche gewährleistet, wobei das Stechtiefenreferenzelement so ausgebildet und angeordnet ist, dass die Referenz-Hautkontaktfläche am Umkehrpunkt der Stechbewegung mit der Haut in Kontakt steht. Der vorgegebene Wert der Stichtiefe wird durch den Abstand in Einstichtiefe zwischen der Referenz-Hautkontaktfläche und der Position der Spitze des Nadelelements an dem Umkehrpunkt der Stechbewegung bestimmt.

Das Stechtiefenreferenzelement mit der Referenz-Hautkontaktfläche befindet sich zu dem Zeitpunkt, zu dem die Spitze des Nadelelements in der Vortriebsphase der Stechbewegung die Referenz-Hautkontaktfläche passiert und in eine mit dieser in Kontakt stehenden Hautoberfläche eindringt, in einer definierten stationären Position in Bezug auf den Umkehrpunkt der Stechbewegung. Außerdem ist das Stechtiefenreferenzelement derartig ausgebildet und angeordnet, dass eine Deformationsstabilisierung der gegen sie gedrückten Hautoberfläche erreicht wird. Durch diese Deformationsstabilisierung wird bewirkt, dass die Reproduzierbarkeit der Stichtiefe nicht in einem praktisch störenden Ausmaß dadurch beeinträchtigt wird, dass die Haut lokal deformiert ("eingedellt") wird, wenn die Spitze der Lanzette in sie eindringt.

Die Erfindung befasst sich primär mit dem Problem, die tatsächlich für die Stichtiefe relevante Position der Hautoberfläche möglichst gut reproduzierbar zu definieren und gleichzeitig einen zuverlässig ausreichenden Blutaustritt ohne zusätzliches "Melken" des Fingers zu gewährleisten. Sie zeichnet sich insbesondere dadurch aus, dass folgende zwei Maßnahmen miteinander kombiniert werden:
a) Das Stechtiefenreferenzelement wird - im Gegensatz zu den vorstehend erörterten bekannten Stechsystemen - zum Zeitpunkt des Einstichs, also wenn die Spitze des Nadelelementes in der Vortriebsphase der Stechbewegung die Referenz-Hautkontaktfläche passiert, nicht gemeinsam mit der Nadel bewegt, sondern es befindet sich in einer fixen (stationären) Position relativ zu dem Umkehrpunkt des Stechgerätes. Vorzugsweise bleibt das Referenzelement mindestens bis zu dem Zeitpunkt, zu dem das Nadelelement den Umkehrpunkt seiner Stechbewegung erreicht, in der stationären Position. Besonders bevorzugt befindet sich das Stechtiefenreferenzelement während der gesamten Vortriebsphase der Stechbewegung des Nadelelementes, also vom Auslösen der Stechbewegung bis zum Erreichen des Umkehrpunktes, in seiner definierten stationären Position.
   Wenn der Stechantrieb in dem Gehäuse des Stechgerätes starr fixiert ist, ist die Position des Umkehrpunktes der Stechbewegung, bezogen auf das Gehäuse, fix und folglich das Referenzelement auch bezogen auf das Gehäuse stationär. Gemäß einer bevorzugten Ausführungsform der Erfindung, die weiter unten noch näher erläutert wird, ist jedoch vorgesehen, dass das Stechtiefenreferenzelement und mindestens ein Teil des Stechantriebs derartig gemeinsam federnd gelagert sind, dass sie bei Druck auf die Referenz-Hautkontaktfläche eine synchrone Bewegung mit gleichbleibendem Abstand in Einstichrichtung ausführen können. In diesem Fall ist das Stechtiefenreferenzelement mit der Hautkontaktfläche bezogen auf das Gehäuse nicht stationär, jedoch weiterhin stationär bezogen auf den Umkehrpunkt der Stechbewegung. Auch in diesem Fall befindet sich das Stechtiefenreferenzelement jedoch während der gesamten Vortriebsphase der Stechbewegung des Nadelelementes auch relativ zu dem Gehäuse in einer praktisch stationären Position, weil eventuelle durch Variation des Druckes auf die Referenz-Hautkontaktfläche verursachte Bewegungen des Referenzelementes (und des mit diesem gemeinsam federnd gelagerten Teils des Stechantriebs) sehr viel langsamer als die Stechbewegung in ihrer Vortriebsphase sind.
b) Das Stechtiefenreferenzelement ist derartig ausgebildet und angeordnet, dass beim Andrücken der Haut an die Referenz-Hautkontaktfläche eine Deformationsstabilisierung erreicht wird. Mit diesem Begriff wird eine Straffung der Haut bezeichnet, durch die verhindert wird, dass die Reproduzierbarkeit der Stichtiefe durch eine beim Einstechen der Spitze des Nadelelementes in die Haut auftretende Hautdeformation in störender Weise beeinträchtigt wird. Dies wird insbesondere dadurch gewährleistet, dass eine hinreichend hohe, "deformationsstabilisierende" Druckkraft während des Einstichs zwischen der Referenz-Hautkontaktfläche und der Haut wirkt.

In mehreren oben diskutierten Dokumenten des Standes der Technik wird es als vorteilhaft angesehen, wenn das Stechtiefenreferenzelement gemeinsam mit dem Nadelelement in Richtung auf die Haut bewegt wird und mit einem erheblichen Impuls auf die Haut auftrifft. Im Rahmen der vorliegenden Erfindung wurde festgestellt, dass bei diesen bekannten Konstruktionen das Auftreffen des Referenzelementes auf die Haut eine teilelastische Stoßwirkung verursacht, durch die der Einstichschmerz erhöht wird. Dieser erhöhte Schmerz wird nach den vorliegenden Erkenntnissen nicht etwa direkt durch das Auftreffen des Referenzelementes verursacht, sondern ist eine indirekte Wirkung, die mit einer durch den teilelastischen Stoß verursachten Unruhe der Lanzettenbewegung zusammenhängt. Dieses Problem wird bei der vorliegenden Erfindung durch die stationäre Positionierung des Stechtiefenreferenzelementes vermieden.

Eine wesentliche Verbesserung gegenüber der oben diskutierten EP 1669028 wird dadurch erreicht, dass zum Zeitpunkt des Einstichs Bedingungen herrschen, die einerseits die erläuterte Deformationsstabilisierung bewirken, andererseits aber die Blutexpression behindern. Insbesondere wird - im Gegensatz zu der vorbekannten Konstruktion - die Kraft, mit der die Haut zum Zeitpunkt des Einstichs gegen die Referenz-Hautkontaktfläche drückt ("Druckkraft") auf einen hinreichend hohen deformationsstabilisierenden Wert eingestellt wird. Dies kann insbesondere auf zweierlei Weise realisiert werden:
a) Mittels eines Kraftsensors, der die Druckkraft misst. Die gemessene Druckkraft kann dem Benutzer auf einem Display angezeigt werden, so dass dieser den Stich auslöst, wenn die Druckkraft zwischen einem Mindest- und einem Höchstwert liegt. Selbstverständlich kann die Auslösung auch automatisch erfolgen. Der Drucksensor ist vorteilhaft mit dem Stechtiefenreferenzelement gekoppelt. Beispielsweise kann das Stechtiefenreferenzelement einen Drucksensor aufweisen, mittels dessen der Anpressdruck ermittelt wird. Der Sensor kann mechanisch, elektrisch oder optisch ausgeführt sein. Es sind auch weitere sensorische Einrichtungen denkbar, die den Anpressdruck der Haut an die Referenz-Hautkontaktfläche ermitteln. Sie müssen nicht zwangsläufig mit dem Stechtiefenreferenzelement gekoppelt sein.
b) Das Stechtiefenreferenzelement kann zur Gewährleistung der gewünschten hautstabilisierenden Druckkraft beweglich gelagert sein, wobei es mittels einer Federeinrichtung in Einstichrichtung derartig gegen einen gehäusefesten Anschlag gedrückt wird, dass es beim Andrücken der Haut gegen seine Referenz-Hautkontaktfläche von dem Anschlag abgehoben und gegen die Kraft der Federeinrichtung entgegen der Einstichrichtung so nach hinten verschoben wird, dass die auf die Haut wirkende Druckkraft der Federkraft der Federeinrichtung entspricht. Dabei sollte die Kraft der Federeinrichtung möglichst stabil sein. Dies lässt sich beispielsweise durch eine vorgespannte Metallfeder mit einer im Bewegungsbereich des Stechtiefenreferenzelementes flachen Federkennlinie, aber beispielsweise auch mittels einer Gasfeder erreichen.

Da die deformationsstabilisierende Wirkung von einer Mehrzahl von Faktoren, darunter den Dimensionen der beteiligten Bauteile des Stechgerätes, abhängt, ist es nicht möglich, exakte allgemein gültige Angaben über die erforderliche Druckkraft zu machen. In der Praxis hat sich jedoch gezeigt, dass die gewünschte Wirkung bei einer Druckkraft von mindestens 2 N, vorzugsweise mindestens 3 N eintritt. Andererseits sollte die Druckkraft aber auch nicht zu hoch sein. Bevorzugt beträgt ihr Höchstwert 7 N, wobei Werte von höchstens 5 N besonders bevorzugt sind.

Bei der Benutzung des erfindungsgemäßen Stechsystems drückt der Benutzer die Haut seines Körperteils, vorzugsweise seines Fingers, gegen eine Gehäuse-Hautkontaktfläche. Dabei wird die Haut gegen die Referenz-Hautkontaktfläche gedrückt. Durch den Anpressdruck gegen die Referenz-Hautkontaktfläche wird die Haut gestrafft, bevor der Einstich der Spitze des Nadelelements in die Haut erfolgt. Ein Eindellen der Haut wird verhindert. Das Schmerzempfinden des Benutzers beim Einstich wird verringert und die Reproduzierbarkeit der Stichtiefe verbessert.

Die viskoelastischen Eigenschaften der Haut spielen beim Einstich bei dem erfindungsgemäßen Stechsystem keine Rolle mehr.

Die erfindungsgemäße stationäre Positionierung des Stechtiefenreferenzelementes ist nicht so zu verstehen, dass es permanent an dem Gehäuse des Stechgerätes fixiert ist. Vielmehr ist es mittels eines Referenzelementantriebs beweglich. Der Referenzelementantrieb ist so ausgebildet, dass das Referenzelement mindestens während eines Teils der Rückführungsphase der Stechbewegung des Nadelelementes von der Haut wegbewegt wird. Dadurch werden optimale Expressionsbedingungen erreicht.

Im Rahmen der vorliegenden Erfindung wurde festgestellt, dass es vorteilhaft ist, das Stechsystem so zu konstruieren, dass zwei klar differenzierte Zustände gewährleistet werden, nämlich ein Einstichzustand und ein Expressionszustand:
- In dem Einstichzustand werden kompromisslos optimale Einstichbedingungen hergestellt, so dass eine ausgezeichnete Reproduzierbarkeit der Stichtiefe in Verbindung mit einem sehr geringen Schmerz erreicht wird. Die Einstichbedingungen sind gezielt so gewählt, dass - insbesondere wegen der herrschenden relativ hohen Druckkraft - keine Expression stattfindet, also noch kein Blut austritt.
- Danach wird das Stechtiefenreferenzelement (mindestens) so weit von der Haut, die sich in die Gehäuseöffnung hineinwölbt, weg bewegt, dass die Expressionshilfe optimal wirkt.

Das Stechtiefenreferenzelement kann nicht nur nach, sondern auch vor dem Einstich mittels des Referenzelementantriebs bewegt werden. Bevorzugt ist die Bewegung jedoch beendet, bevor die Haut an die Gehäuse-Hautkontaktfläche gedrückt wird, wobei eine Signaleinrichtung vorgesehen sein kann, durch die dem Benutzer angezeigt wird, dass das Stechgerät zum Andrücken bereit ist. Vorteilhaft ist ein Stechsystem mit einer Auslösesperre, die derart ausgebildet und angeordnet ist, dass der Stechvorgang des Nadelelements erst dann ausgelöst werden kann, wenn die Haut mit einem bestimmten Mindestanpressdruck an der Referenz-Hautkontaktfläche anliegt.

Das erfindungsgemäße Stechsystem ist für ein "one-step-handling" geeignet. Darunter wird verstanden, dass der Benutzer das Stechsystem nur einmal an den Finger ansetzen muss, während von dem System mehrere Verfahrensschritte durchgeführt werden, wie beispielsweise das Erzeugen der Wunde im Finger, das Herausdrücken des Blutes aus dem Finger und das Sammeln des Blutes nach dem erfolgten Stich.

Zu den "one-step-handling" Systemen gehören insbesondere Stechsysteme mit kanülenartigen Nadelelementen, bei denen die Nadel nach Erreichen des Umkehrpunktes nicht vollständig aus der Haut herausgezogen wird, sondern über einen definierten Zeitraum in der Haut verbleibt. Ein solches System ist in der EP 1797822, der US 2004/059256 A1 und der internationalen Patentanmeldung PCT/EP 2006/001922 beschrieben. Darin ist auch ein Stechsystem offenbart, in dem die gewünschte Stechbewegung des Nadelelements mittels Steuerkurve gesteuert wird. Der Inhalt dieser Anmeldungen wird durch Referenzierung zum Inhalt der vorliegenden Anmeldung gemacht.

Das erfindungsgemäße System hat ein Nadelelement, das einen Kapillarkanal aufweist. Durch diesen kann nach dem Einstich in die Haut Blut gesammelt werden. Ein derartiges System wird noch näher beschrieben.

In einer bevorzugten Ausgestaltung ist das Stechgerät mehrfach verwendbar. Es schließt eine Halterung ein, mittels der eine Stecheinheit auswechselbar mit dem Stechantrieb gekoppelt werden kann. Dabei ist die Referenz-Hautkontaktfläche des Stechtiefenreferenzelements an einem disposiblen Element ausgebildet, das zur einmaligen Verwendung vorgesehen ist. Die Stecheinheit kann dann aus einem Nadelelement bzw. einer Lanzetten und einem Stechtiefenreferenzelement bestehen.

Die Erfindung wird nachfolgend anhand von in den Figuren dargestellten bevorzugten Ausführungsformen näher erläutert. Die darin dargestellten Besonderheiten können einzeln oder in Kombination verwendet werden, um bevorzugte Ausgestaltungen der Erfindung zu schaffen. Die beschriebenen Ausführungsformen stellen keine Einschränkung der Allgemeinheit des in den Ansprüchen definierten Gegenstands dar. Es zeigen:
- Fig. 1: eine schematische Schnittdarstellung eines Teils um eine Gehäuseöffnung einer ersten Ausführungsform eines Stechsystems;
- Fig. 2: eine Figur 1 entsprechende Darstellung in sieben Benutzungspositionen a bis g des Stechsystems;
- Fig. 3: eine Antriebseinheit bestehend aus Spannrotor und Antriebsrotor;
- Fig. 4: eine perspektivische Ansicht einer Antriebsmechanik mit einer Antriebseinheit, einem Koppelmechanismus und einer Stecheinheit;
- Fig. 5: eine weitere perspektivische Ansicht der Antriebseinheit aus Figur 4;
- Fig. 6: eine der Figur 2 entsprechende Darstellung der Positionen a bis i der Antriebseinheit;
- Fig. 7: die drei sich ergebenden Steuerkurven der Antriebsmechanik sowie die resultierende Kurve des Nadelelements über eine 360°-Drehung;
- Fig. 8: die Antriebseinheit bei eingestellter minimaler und maximaler Stichtiefe des Nadelelements;
- Fig. 9: ein als Verschluss ausgebildetes Stechtiefenreferenzelement in drei Verschlusssteltungen a bis c;
- Fig. 10: zwei Verschlussstellungen des Verschlusses aus Figur 9 in Seitenansichten a und b;
- Fig. 11a,b: ein alternatives Stechtiefenreferenzelement mit mehreren Bauteilen;
- Fig. 12a,b: eine weitere Ausführungsform eines Stechtiefenreferenzelements;
- Fig. 13a,b: eine weitere Ausführungsform des Stechtiefenreferenzelements; und
- Fig. 14: eine alternative Ausführungsform eines Stechgeräts mit integrierter Analyseeinheit.

Die Ortsangaben "vorne" und "hinten" beziehen sich auf die Einstichrichtung. So ist das vordere Ende des Stechsystems das Ende, an dem die Lanzette oder Nadel bei Bewegung in Einstichrichtung aus dem Referenzelement heraustritt.

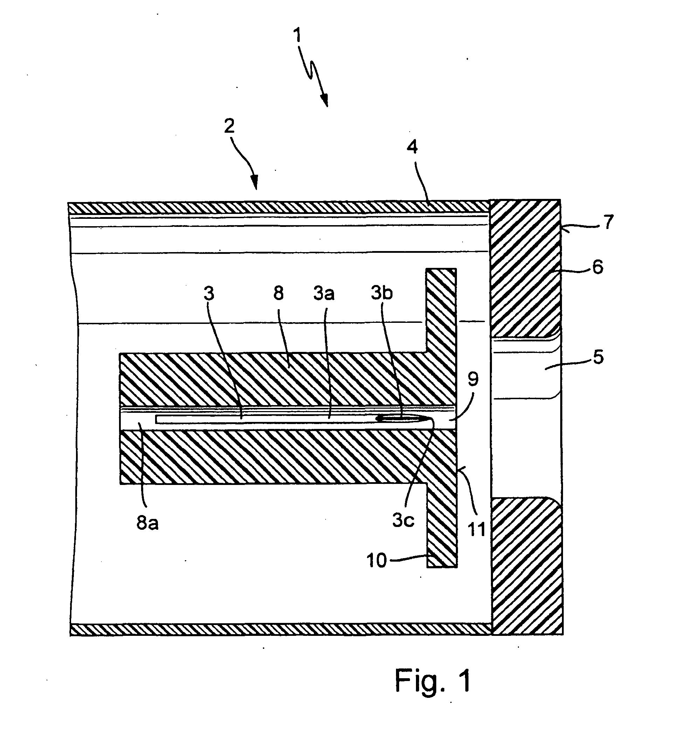

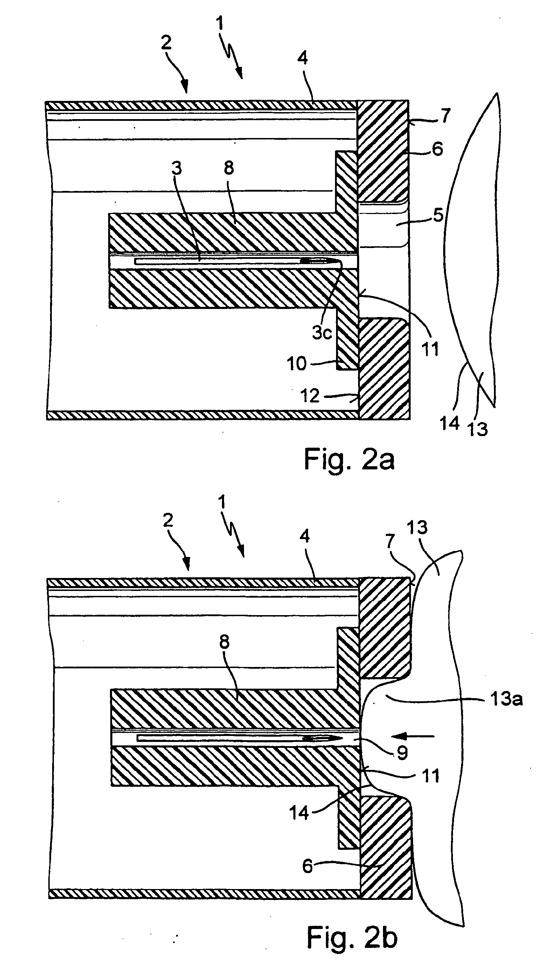

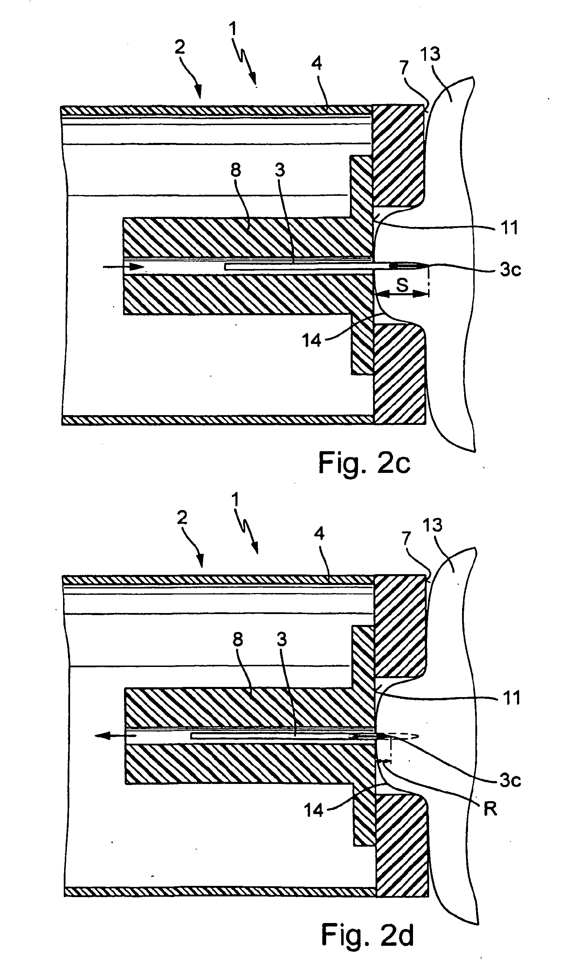

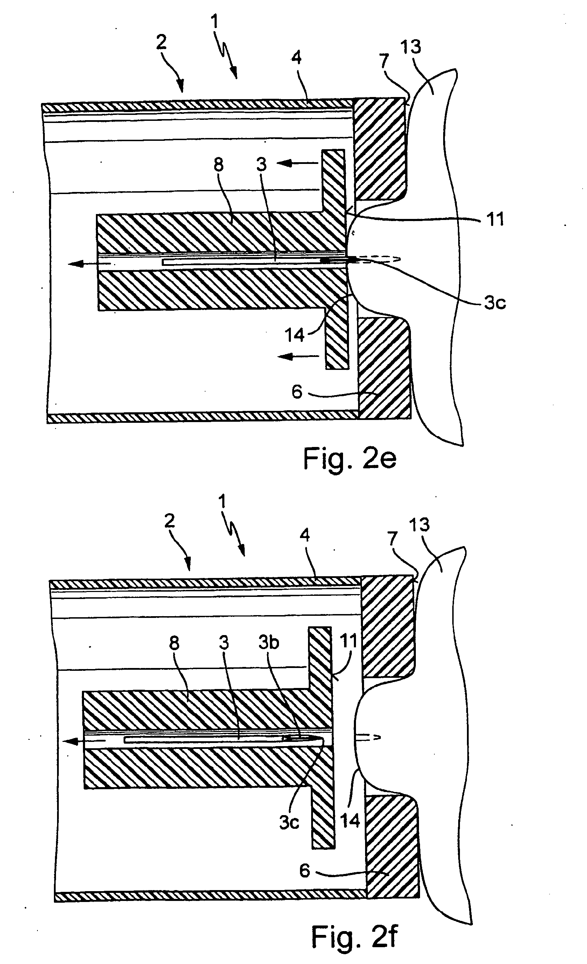

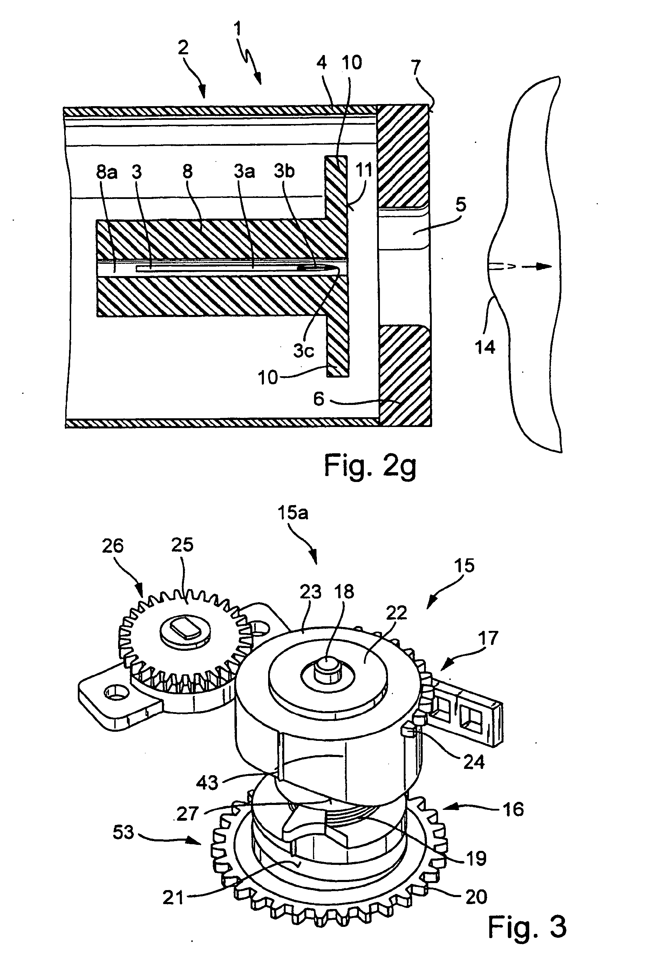

Die Figuren 1 und 2 zeigen schematisch das in Einstichrichtung vorne liegende Ende eines in der Gesamtheit nicht dargestellten Stechsystems 1. Das Stechsystem 1 umfasst ein ebenfalls nicht vollständig dargestelltes Stechgerät 2 und ein Nadelelement 3. Das Stechgerät 2 hat ein Gehäuse 4 mit einer Gehäuseöffnung 5, die an der vorderen Wandung 6 des Stechgeräts angeordnet ist.

Da die expressionsfördernde Wirkung von mehreren Faktoren abhängt, lassen sich keine scharfen, für alle Anwendungsfälle richtigen Grenzwerte für die Mindestweite der Gehäuseöffnung 5 angeben. In der Praxis hat sich jedoch eine minimale Weite der Gehäuseöffnung 5 als vorteilhaft erwiesen, die (für den Fall einer kreisförmigen Öffnung) einem Durchmesser von mindestens 4 mm, besonders bevorzugt mindestens 6 mm entspricht. Vorzugsweise ist die Gehäuseöffnung 5 kreisrund und hat einen Durchmesser von 4 bis 11 mm, bevorzugt von 6 bis 8 mm. Auch wenn sie nicht exakt kreisförmig ist, sollte ihre Querschnittsfläche der Fläche eines Kreises mit den genannten Durchmesserwerten entsprechen. Die Öffnung ist jedenfalls so groß, dass sich die Haut eines Fingers oder eines anderen Körperteils in die Öffnung wölben kann. Eine die Gehäuseöffnung 5 umschließende Gehäuse-Hautkontaktfläche 7 wird bei der Benutzung an die Haut eines Körperteils gedrückt.

Das Nadelelement 3 ist als Kanüle 3a ausgebildet, die einen Kapillarkanal 3b aufweist und deren vorderes Ende derart abgeschrägt ist, dass eine Spitze 3c gebildet wird. Besonders bevorzugt ist das als Sampler bezeichnete Nadelelement 3 aus einem flachen Blech hergestellt und weist einen Kapillarkanal 3b, bevorzugt einen einseitig offenen Kapillarkanal, auf, durch den eine Körperflüssigkeit, insbesondere Blut, aus der Haut aufgenommen werden kann.

Das Nadelelement 3 ist relativ zu einem Stechtiefenreferenzelement 8 präzise geführt, beispielsweise wie in Figur 1 dargestellt, in einem Führungskanal 8a des Stechtiefenreferenzelements 8, das vorne, also in Einstichrichtung des Nadelelements 3, eine Elementöffnung 9 aufweist. Die Elementöffnung 9 ist deutlich kleiner als die Gehäuseöffnung 5. Das Stechtiefenreferenzelement 8 hat in dem hier gezeigten Beispiel an seinem vorderen Ende zwei Anschläge 10. Die Anschläge 10 können selbstverständlich auch an einer anderen Stelle des Referenzelementes 8 ausgebildet sein. Ebenfalls am vorderen Ende des Stechtiefenreferenzelementes 8 ist eine Referenz-Hautkontaktfläche 11 angeordnet, die dazu eingerichtet ist, mit der Haut des Körperteils in Kontakt zu gelangen.

Figur 1 stellt die Ausgangsposition des Stechsystems 1 dar, bei der das Stechtiefenreferenzelement 8 in einer Ruheposition so angeordnet ist, dass es von dem Rand des Gehäuses 4 beabstandet ist. Bevor das Stechsystem 1 verwendet werden kann, wird das Stechtiefenreferenzelement 8 in seine definierte stationäre Position gebracht, in der es auch verbleibt, wenn die Spitze 3a des Nadelelements 3 in der Vortriebsphase der Stechbewegung die Referenz-Hautkontaktfläche 11 passiert und aus dem Stechtiefenreferenzelement 8 heraustritt. Diese als Startposition bezeichnete Konfiguration ist in Figur 2a näher veranschaulicht.

Die Figuren 2a bis f zeigen verschiedene Benutzungspositionen des Stechsystems 1. In den Figuren wird der Ablauf der Stechbewegung in einzelnen Phasen dargestellt, wobei im Anschluss an das Erreichen des Umkehrpunkts der Stechbewegung die Spitze 3a der Nadel 3 auf eine Resteindringtiefe bzw. Reststichtiefe zurückgezogen wird. Von hier an bewegen sich das Nadelelement 3 und die Referenz-Hautkontaktfläche 11 gemeinsam unter Beibehaltung des Überstands des Nadelelements 3 über die Referenz-Hautkontaktfläche 11 von der Haut weg und entlasten diese derart, dass Blut austreten kann. Der Kapillarkanal 3b des Nadelelements 3 nimmt das Blut auf. Diese Bewegungsphase wird als Sammelphase bezeichnet.

In der Startposition des Stechsystems 1 (Figur 2a) befindet sich das Stechtiefenreferenzelement 8 in seiner definierten stationären Position in Bezug auf das Gehäuse 4. Die definierte stationäre Position des Stechtiefenreferenzelements 8 kann auch auf die Gehäuse-Hautkontaktfläche 7 bezogen werden. Die stationäre Position wird beispielsweise dadurch erreicht, dass das Stechtiefenreferenzelement 8 mit seinem Anschlag 10 an einem korrespondierenden Gehäuseanschlag 12 an der Gehäuseöffnung 5 zusammenwirkt. Die stationäre Position der Referenz-Hautkontaktfläche 11 wird nun durch den Kontakt mit des Anschlags 10 mit dem Gehäuseanschlag 12 eindeutig definiert. Es ist klar, dass die Verwendung von korrespondierenden Anschlägen nur eine Möglichkeit ist, um das Stechtiefenreferenzelement 8 in der definierten stationären Position anzuordnen. Das Stechtiefenreferenzelement 8 kann auch beweglich gelagert sein, wobei es mittels einer Feder gegen den Anschlag 12 wirken kann. Wichtig dabei ist einzig, dass die stationäre Position derart eingehalten wird, dass die durch das Stechtiefenreferenzelement 8 eingestrellte und festgelegte Stichtiefe des Nadelelements 3 zuverlässig gewährleistet werden kann.

In der stationären Position des Stechtiefenreferenzelement 8 ist das Nadelelement 3 derart angeordnet, dass seine Spitze 3c hinter der Referenz-Hautkontaktfläche 11 angeordnet ist. In dem gezeigten Beispiel befindet sich die Spitze 3c ca. 3 bis 4 mm hinter der Referenz-Hautkontaktfläche 11.

Das Stechtiefenreferenzelement 8 des Stechsystems 1 ist bevorzugt also bereits in seiner stationären Position angeordnet, bevor das Stechsystem 1 mit einem Körperteil 13 in Kontakt kommt, also bevor die Haut 14 des Fingers 13 an die Gehäuse-Hautkontaktfläche 7 angedrückt wird.

In dem Moment, in dem das Stechsystem 1 gegen den Finger 13 gedrückt wird, wölbt sich die Haut 14 des Fingers in die Gehäuseöffnung 5 des Stechgeräts 2 hinein und drückt an die Referenz-Hautkontaktfläche 11 an, Figur 2b. Die Haut 14 liegt also sowohl an der Referenz-Hautkontaktfläche 11 als auch an der Gehäuse-Hautkontaktfläche 7 an. Der Anpressdruck der Haut an die Gehäuse-Hautkontaktfläche 7 liegt im Bereich von etwa 5 bis 10 N. Die eingewölbte Haut drückt mit ca. 3 N gegen die Referenz-Hautkontaktfläche 11. Diese Druckkraft wird auch bei einem federbelastet gelagertem Stechtiefenreferenzelement 8 eingehalten.

Die Gehäuse-Hautkontaktfläche 7 hat dabei die Aufgabe, den Finger 13 derart zu quetschen, dass in den ausgewölbten Teil 13a des Fingers 13 das Blut hineingedrückt wird. Die Referenz-Hautkontaktfläche 11 begrenzt nicht nur die Auswölbung des Fingers 13, sondern strafft zusätzlich die Haut 14 im Bereich der Elementöffnung 9 des Stechtiefenreferenzelements 8. Somit wird in dem erfindungsgemäßen Stechsystem zum einen das Blut in den Bereich des Körperteils gedrückt, in dem eine Wunde erzeugt werden soll. Zum anderen wird das typischerweise beim Eindringen der Nadel verursachte Eindellen der Haut in diesem Bereich durch die Straffung der Haut verhindert.

Nachdem das Stechsystem 1 an dem Finger 13 anliegt, kann der Stich ausgelöst werden. Das Nadelelement 3 bewegt sich dann in Einstichrichtung und durchstößt mit seiner Spitze 3c die Haut 14 des Fingers 13, wie in Figur 2c dargestellt ist. Dabei ragt die Spitze 3c um die eingestellte Stichtiefe S von ca. 1,5 bis 2,5 mm in die Haut 14 hinein. Diese als Umkehrpunkt definierte Position wird nach ca. 1 msec. erreicht.

Anschließend wird das Nadelelement 3 entgegen der Einstichrichtung bewegt, bis seine Spitze 3c auf eine definierte Reststichtiefe R zurückgezogen ist, die ca. 0,5 mm beträgt und nach etwa 2 msec erreicht wird. Das Stechtiefenreferenzelement 8 bleibt mit seiner Referenz-Hautkontaktfläche 11 in seiner stationären Position und liegt weiterhin an der Haut 14 an. Die bis hierher ablaufenden beiden Teilabschnitte der Stechbewegung, also der Einstich bis zum Erreichen des Umkehrpunktes der Stechbewegung an der Stichtiefe S und der Rückzug des Nadelelements 3 bis zur Reststichtiefe R, sind bevorzugt deutlich schneller als die nachfolgenden Teilabschnitte der Rückführungsphase der Stechbewegung. Während dieser schnellen Bewegung (im Millisekundenbereich), insbesondere während des ersten Teilabschnitts der Rückführungsphase, bleibt die Referenz-Hautkontaktfläche 11 bevorzugt in ihrer stationären Position stehen (Figur 2d).

In dem sich anschließenden Teilabschnitt der Rückführungsphase, Figur 2e, wird das Stechtiefenreferenzelement 8 entgegen der Einstichrichtung bewegt. Vorzugsweise wird während dieses Teils der Rückführungsphase das Stechtiefenreferenzelement 8 gemeinsam mit dem Nadelelement 3 bewegt. Diese Bewegung kann mit unterschiedlichen Geschwindigkeiten vonstatten gehen.

In einer vorteilhaften Ausgestaltung werden jedoch das Stechtiefenreferenzelement 8 und das Nadelelement 3 auf diesem Teil der Rückführungsphase so synchron bewegt, dass die relative Position zwischen der Spitze 3c des Nadelelements 3 und der Referenz-Hautkontaktfläche 11 des Stechtiefenreferenzelements 8 unverändert bleibt. Somit wird also die Reststichtiefe R beibehalten, während das Stechtiefenreferenzelement 8 von der Haut 14 wegbewegt wird. Dadurch kommt es zu einem Entspannen der Haut, die aufgrund ihrer Viskoelastizität dem Stechtiefenreferenzelement 8 folgt. Der Druck des Stechtiefenreferenzelement 8 auf die Haut 14 in dem Bereich um den Einstich wird jedoch reduziert, so dass ein Blutfluss aus der Wunde ermöglicht wird.

Vorzugsweise wird das Stechtiefenreferenzelement 8 also in der Art aus seiner stationären Position relativ zur Gehäuse-Hautkontaktfläche 7 bewegt, dass die Referenz-Hautkontaktfläche 11 mit der Haut 14 in Kontakt bleibt, wobei die Bewegung stattfindet, bevor die Spitze 3c des Nadelelements 3 vollständig aus der Haut 14 herausgezogen ist.

Wenn die Spitze 3c des Nadelelements 3 die Haut 14 nach ca. 0,5 bis 3 sec zuverlässig verlassen hat, wird das Nadelelement 3 des Stechtiefenreferenzelement 8 soweit zurückgezogen, dass sich die Spitze 3c hinter der Referenz-Hautkontaktfläche 11 befindet. Das Nadelelement 3 und das Stechtiefenreferenzelement 8, das ebenfalls soweit entgegen der Einstichrichtung bewegt wurde, dass die Haut 14 nicht mehr an der Referenz-Hautkontaktfläche 11 anliegt, haben dann ihre Ruheposition erreicht. Die mit dem Kapillarkanal 3b gewonnene Probe kann nun an eine Testchemie, beispielsweise an einen Probenträger, übergeben werden. Dabei fließt das Blut aus dem Kapillarkanal 3b des Nadelelements 3 auf den nicht gezeigten Testträger.

Anschließend wird das Stechsystem 1 von der Haut 14 entfernt. Die Haut hat keinen Kontakt mehr zu der Gehäuse-Kontaktfläche 7, wie Figur 2g zu entnehmen ist. Damit ist der Stechvorgang abgeschlossen.

Der Bewegungsablauf der Stechbewegung ist in diesem Fall deutlich verschieden von Stechgeräten, deren Nadelelement 3 auch ohne Kapillarkanal 3b ausgebildet ist. Deren Stechbewegung umfasst lediglich eine schnelle Vortriebsphase bis zum Erreichen des Umkehrpunkts und eine schnelle Rückführungsphase, bis das Nadelelement 3 aus der Haut ausgetreten ist.

Um die in Figur 2 beschriebene Stechbewegung mit Sammelphase zur Aufnahme des austretenden Bluts zu realisieren, sind im Stand der Technik unterschiedliche Antriebskonzepte bekannt. Ein ebenfalls geeignetes Konzept ist in Figur 3 gezeigt. Es weist einen als Antriebseinheit 15 ausgebildeten Stechantrieb 15a auf, die einen Spannrotor 16, einen Antriebsrotor 17 und eine Antriebsfeder 19 sowie einen (hier nicht dargestellten) Motor umfasst. Die beiden Rotoren sind auf einer Achse 18 gelagert. Die zwischen den Rotoren angeordnete Antriebsfeder 19 dient zur Übertragung der Kraft und der Momente von dem Spannrotor 16 auf den Antriebsrotor 17. Der Spannrotor 16 aus Figur 3 hat eine Antriebsverzahnung 20, durch die der Spannrotor 16 von dem Motor der Antriebseinheit 15 angetrieben werden kann.

Die Rotoren der Antriebseinheit 15 werden nur in eine Richtung bewegt, so dass die Rotoren 16,17 nach einer 360°-Umdrehung wieder in ihrer Ausgangsposition angeordnet sind. Ein Zurückdrehen in die entgegengesetzte Richtung ist nicht notwendig. Derartige Antriebseinheiten werden als OWADAC-Antriebe bezeichnet. Ein Beispiel eines solchen Antriebs ist aus der EP 1504718 bekannt. Die dort beschriebene Antriebseinheit ist ebenfalls geeignet, die Stechbewegung nach Figur 2 zu realisieren.

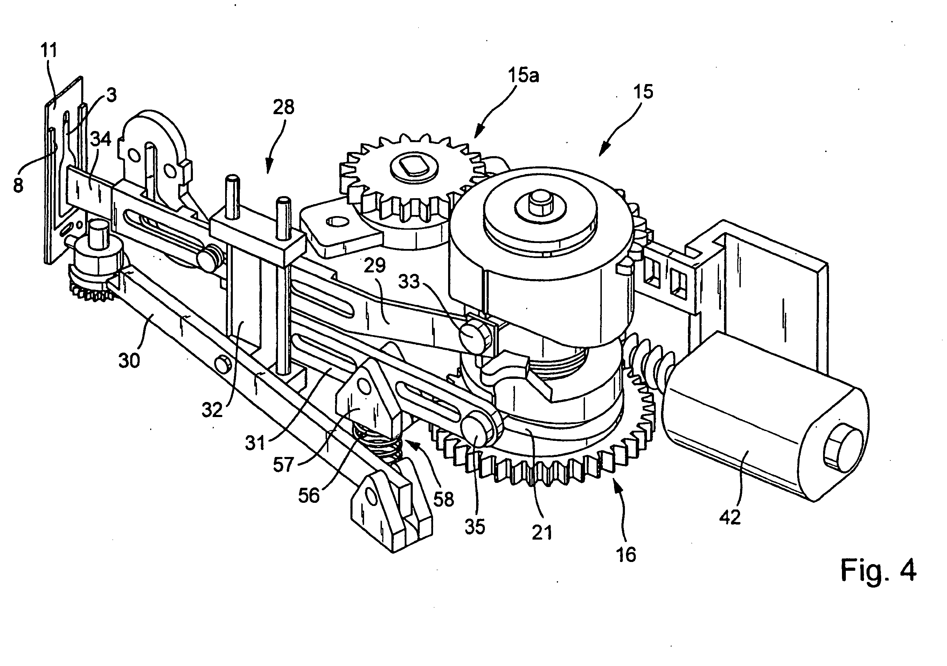

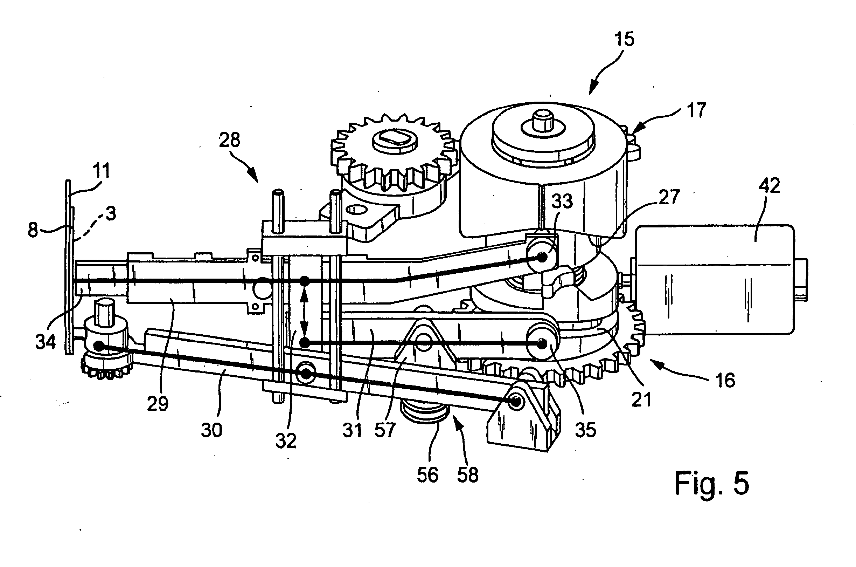

Eine an dem Spannrotor 16 angeordnete Referenzkulisse 21 steuert über einen hier nicht dargestellten Kopplungsmechanismus das Stechtiefenreferenzelement 8 des Stechsystems. Die Referenzkulisse 21 ist vorzugsweise eine enge Nut, in der ein in den Figuren 4 und 5 dargestellter Steuerreiter 35 (englisch: Traveller) zwangsgeführt ist. Die Kulisse kann aber auch eine Steuerungskurve sein, die als Steuerkante oder einseitig offene Nut ausgebildet ist. In ihr kann ein Steuerreiter federbelastet laufen, ohne dass eine Zwangsführung des Steuerreiters entsteht.

Die Spannrotor 16 mit der Referenzkulisse 21 ist Teil eines Referenzelementantriebs 53. Er umfasst daneben auch den Antriebsrotor 17, die Antriebsfeder 18 sowie einen in Zusammenhang mit den Figuren 4 und 5 näher beschriebenen Referenzelementhebel 30. Mittels des Referenzelementantriebs 53 wird das Stechtiefenreferenzelement bewegt. Der Referenzelementantrieb 53 ist unabhängig vom Antrieb des Nadelelements 3. Sie können jedoch mindestens teilweise miteinander gekoppelt sein und auch über gemeinsame Komponente verfügen, wie es in Figur 4 verdeutlich ist. Beispielsweise ist die Antriebsfeder 19 in dem vorliegenden Beispiel eine gemeinsame Komponente der beiden Antriebe. Der Referenzelementantrieb 53 kann jedoch auch elektrisch, elektronisch oder elektromechanisch vom Antrieb des Nadelelements getrennt sein; auch rein mechanische Trennungen sind möglich.

Der Antriebsrotor 17 der Antriebseinheit 15 ist zweiteilig ausgebildet, wobei ein inneres Verstellteil 22 gegenüber einem äußeren Rotorteil 23 axial verstellbar ist. Durch die Verstellung der beiden Teile zueinander wird die Stichtiefe des Nadelelements 3 gegenüber dem Stechtiefenreferenzelement 8 definiert und eingestellt. Eine Stichkulisse 43, die einen Teil des Einstichwegs des Nadelelements und den Umkehrpunkt der Stechbewegung bestimmt, wird je nach Stellung des Verstellteils 22 gegenüber dem Rotorteil 23 teilweise verdeckt. Aus der zweiteiligen Ausbildung des Antriebsrotors 17 ergibt sich auch eine zweiteilige Steuerkurve, die aus der Stichkulisse 43 und der Lanzettenkulisse 27 gebildet wird. Da die zweiteilige Steuerkurve in ihrer Form veränderbar ist, kann sie nicht als geschlossene, zwangsgeführte Bahn ausgeführt werden. Der Steuerreiter 33 am Lanzettenhebel 29, der in der zweiteiligen Steuerkurve geführt wird, wird deshalb über eine (nicht dargestellte) Federkraft dazu gezwungen, der Steuerkante der Steuerkurve zu folgen.

Das Rotorteil 23 des Antriebsrotors 17 weist an einem Teil eines äußeren Umfangs eine Verzahnung 24 auf, die während eines Teils der Drehbewegung mit einem als Zahnrad 25 ausgebildeten Dämpfer 26 so in Eingriff kommt, dass die Bewegung des Antriebsrotors 17 durch den Dämpfer 26 verlangsamt wird.

Das Rotorteil 23 ist an seiner (zum Spannrotor 16 ausgerichteten) Unterseite in Form einer Lanzettenkulisse 27 ausgebildet, um über einen Kopplungsmechanismus die Bewegung des Nadelelements zu bewirken.

Die Figuren 4 und 5 zeigen jeweils in perspektivischer Darstellung eine Antriebsmechanik, in der die Antriebseinheit 15 aus Figur 3 integriert ist. Ein Kopplungsmechanismus 28 umfasst einen Lanzettenhebel 29, den Referenzelementhebel 30 und einen Kopplungshebel 31 sowie einen Lagerbock 32. Der Lanzettenhebel 29, der Referenzelementhebel 30 und der Kopplungshebel 31 sind über den Lagerbock 32 miteinander gekoppelt. Eine axiale Verschiebung des Lagerbocks 32, die durch einen der Hebel bewirkt wird, wird auf die anderen Hebel wenigstens teilweise bzw. zeitweise so übertragen, dass eine gleichzeitige Bewegung des Nadelelements 3 und des Stechtiefenreferenzelements 8 ermöglicht werden kann. Mittels des Kopplungsmechanismus 28 werden die Drehbewegungen der Antriebseinheit 15 auf die aus Nadelelement 3 und Stechtiefenreferenzelement 8 mit Referenz-Hautkontaktfläche 11 bestehende Stecheinheit übertragen.

Ein Steuerreiter 33, der an einem Ende des Lanzettenhebels 29 angeordnet und befestigt ist, fährt die Lanzettenkulisse 27 und die Stichkulisse 43 des Antriebsrotors 17 ab. Die Bewegung des Steuerreiters 33 wird über den Lanzettenhebel 29 auf die Lanzette bzw. das Nadelelement 3 übertragen, das mittels eines Kopplungselements 34 an dem Lanzettenhebel 29 befestigt ist. Auf diese Weise können verschiedene Nadelelemente bzw. Lanzetten mit dem Stechgerät verbunden werden. Insbesondere bei Einmallanzetten ist ein derartiges Kopplungselement 34 notwendig, um das Stechgerät mehrfach zu verwenden.

Der Kopplungshebel 31 wird über einen Steuerreiter 35, der entlang der Referenzkulisse 21 des Spannrotors 16 geführt wird, bewegt. Der Kopplungshebel 31 ist so an dem Lagerbock 32 befestigt, dass eine durch Abfahren der Referenzkulisse 21 hervorgerufene Bewegung des Kopplungshebels 31 eine axiale Verschiebung des Lagerbocks 32 in und entgegen der Einstichrichtung bewirkt. Die axiale Bewegung des Lagerbocks 32 ruft eine Bewegung des mit dem Referenzelementhebel 30 gekoppelten Stechtiefenreferenzelements 8 hervor.

Durch die Kombination der verschiedenen Hebel des Kopplungsmechanismus 28 mit dem Lagerbock 32 ist es möglich, das Nadelelement 3 während eines Teils der Rückführungsphase der Stechbewegung gemeinsam mit dem Stechtiefenreferenzelement 8 derart zu bewegen, dass die gewünschte Reststichtiefe konstant bleibt.

Diese Anordnung ermöglicht es ebenfalls, die Referenz-Hautkontaktfläche 11 gegenüber dem Gehäuse 4 unter einer Hauteindruckkraft, die vorzugsweise ca. 3 N beträgt, einfedern zu lassen, ohne dass die Stichtiefe und/oder die Reststichtiefe in der Sammelphase (Sammeltiefe) beeinflusst werden.

Der Kopplungshebel 31, der den Steuerreiter 35 und den Lagerbock 32 verbindet, ist in einem Lager 57 drehbar gelagert. Das Lager 57 wird von einer als Feder 56 ausgebildeten Federeinrichtung 58 gehalten. Beispielsweise kann die Feder 56 als Metallfeder ausgelegt sein. Hierdurch ergibt sich eine federnde Lagerung des Lagerbocks 32 und somit letztendlich eine federnde Lagerung des Stechtiefenreferenzelements 8 mit der an seinem oberen Ende angeordneten Referenz-Hautkontaktfläche 11.

Das Stechtiefenreferenzelement 8 ist entgegen der Einstichrichtung des Nadelelements 3 beweglich gelagert. Die Feder 56 drückt das Lager 57 durch ihre Vorspannung in Einstichrichtung. Die Auslenkung des Lagers 57 in Einstichrichtung bzw. die Auslenkung des Kopplungsmechanismus 28 in Einstichrichtung kann durch einen Anschlag begrenzt werden. Auch ist es möglich, das Stechtiefenreferenzelement 8 selbst durch einen Anschlag zu begrenzen, so dass der von der Feder 56 ausgeübte Federweg limitiert ist.

Beim Andrücken der Haut gegen die Referenz-Hautkontaktfläche 11 wird das Stechtiefenreferenzelement 8 entgegen der Kraft der Feder 56 entgegen der Einstichrichtung nach hinten verschoben, wobei die Feder 56 zusammengedrückt wird. Die auf die Haut wirkende Druckkraft entspricht dann der Federkraft der Federeinrichtung 58. Auf diese Weise kann eine Begrenzung des maximal zulässigen Drucks auf die Haut erreicht werden.

Den Figuren 4 und 5 ist ebenfalls zu entnehmen, dass das Stechtiefenreferenzelement 8 und das Nadelelement 3 jeweils disposible Elemente sein können. Bevorzugt werden sie gemeinsam ausgetauscht, wobei der Stechantrieb 15 und der Kopplungsmechanismus 28 mehrfach verwendet werden können.

In den Figuren 4 und 5 ist ein Stechtiefenreferenzelement 8 ohne eine Elementöffnung 9 dargestellt. Es kann beispielsweise ein im wesentlichen planes Element sein, das auch gleichzeitig die Funktion eines Analyseelementes erfüllen kann und dessen obere Kante die Referenz-Hautkontaktfläche 11 bildet. Das Nadelelement 3 wird dann während der Stechbewegung an dem Stechtiefenreferenzelement 8 entlang geführt und passiert die Referenz-Hautkontaktfläche 11. Der minimale Abstand, also die kleinste Entfernung, zwischen der Referenz-Hautkontaktfläche 11 und dem Nadelelement 3 beträgt dabei höchstens 1 mm, bevorzugt höchstens 0,5 mm. Durch den relativ geringen Abstand wird gewährleistet, dass das Nadelelement 3 in einen Bereich der Haut einsticht, der von der Referenz-Hautkontaktfläche 11 gestrafft wird.

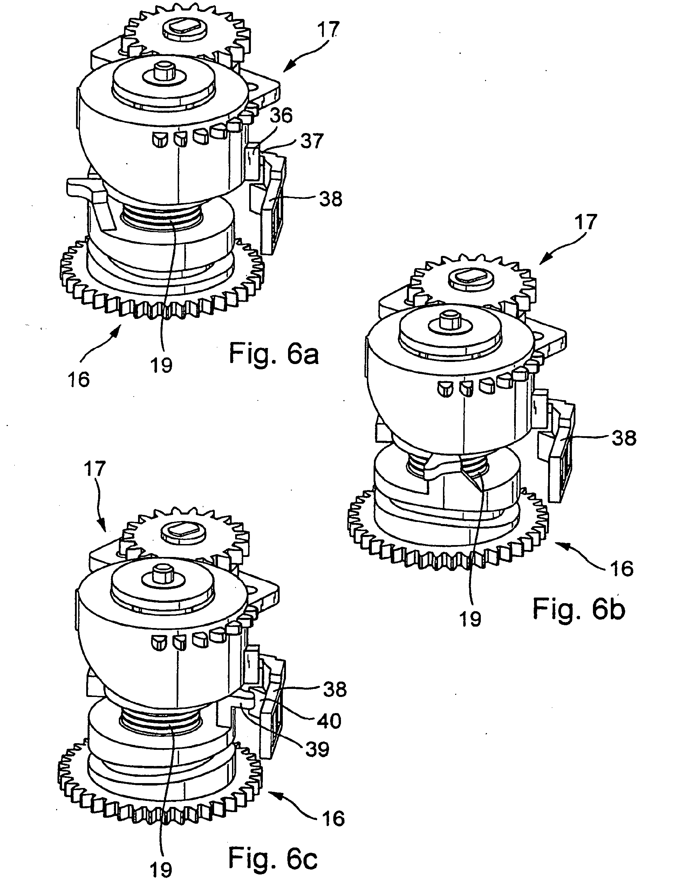

Der Bewegungsablauf der Antriebseinheit 15 zur Realisierung der in Figur 2 beschriebenen Stechbewegung ist in den Figuren 6a bis i detailliert gezeigt. Dabei werden verschiedene Stellungen des Spannrotors 16 in Bezug auf den Antriebsrotor 17 dargestellt.

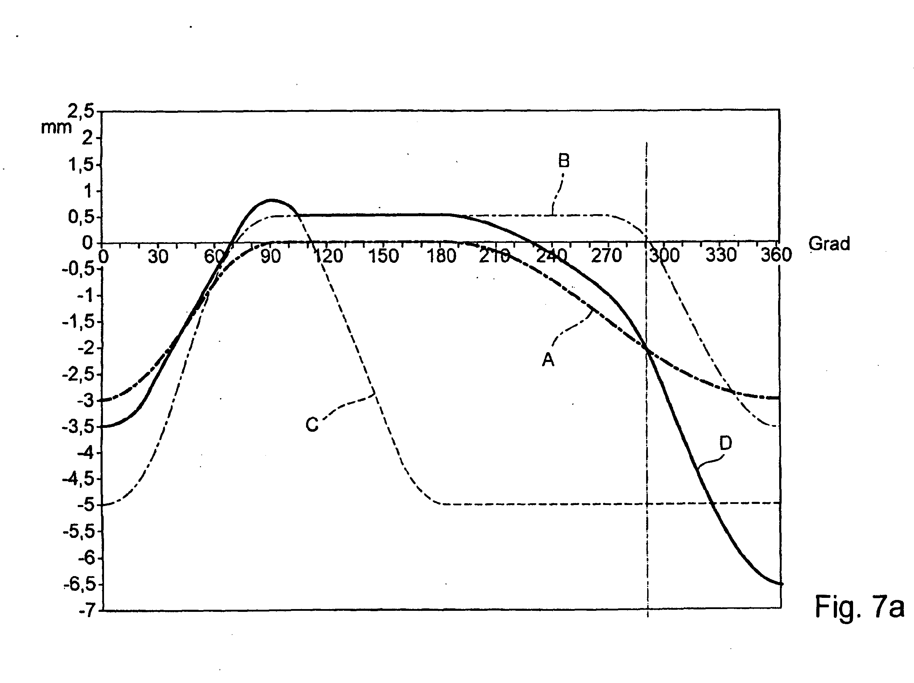

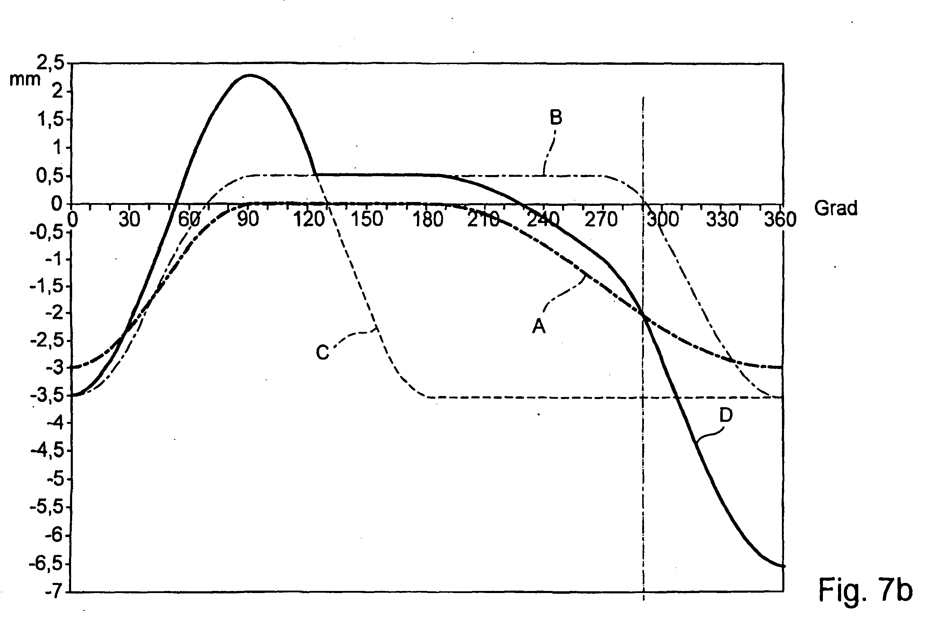

Die verschiedenen Winkelpositionen der beiden Rotoren über eine 360°-Umdrehung sind in den Grafiken nach Figur 7a und b verdeutlicht, wobei die Drehwinkel der Rotoren in Grad über die X-Achse aufgetragen sind. Die Figuren 7a und b zeigen eine geometrische Darstellung der durch die Kulissen des Spannrotors 16 und des Antriebsrotors 17 hervorgerufenen Hübe des mit der Stecheinheit verbundenen Endes des Lanzettenhebels 29 bzw. des Referenzelementhebels 30. Die Kurve A zeigt den Hub des Referenzelementhebels 30, was der Position des Stechtiefenreferenzelement 8 entspricht. Die Kurve B zeigt den durch die Lanzettenkulisse 27 hervorgerufenen Hub; die Kurze C den durch die Stichkulisse 43 hervorgerufenen Hub. Die Kurve D ist die resultierende Kurve (die den Hub bzw. die Stechbewegung des Nadelelements 3 repräsentiert).

Figur 6a zeigt die Ausgangsposition der beiden Rotoren 16,17, die mit 0° bezeichnet ist. Der Antriebsrotor 17 liegt mit einem Haltenocken 36 an einen Rasthaken 37 einer Verrastung 38 an, so dass die Bewegung des Antriebsrotors 17 in Pfeilrichtung, also entgegen dem Uhrzeigersinn, gesperrt ist.

Der Spannrotor 16 wird nun durch einen in den Figuren 4 und 5 dargestellten Antriebsmotor 42 entgegen dem Uhrzeigersinn gedreht. Dabei wird die Antriebsfeder 19 zwischen dem Spannrotor 16 und dem Antriebsrotor 17 langsam über ihre Verspannung hinaus gespannt (Figur 6b). Gleichzeitig wird das über den Kopplungsmechanismus 28 gekoppelte Stechtiefenreferenzelement 8 in seine definierte stationäre Position bewegt (vgl. Figur 2a). Das Nadelelement 3 wird mit dem Stechtiefenreferenzelement 8 gemeinsam bewegt, so dass die Relativposition der beiden zueinander konstant bleibt. Etwa nach einer 90°-Drehung des Spannrotors 16 (Kurve A, Figur 7a,b) hat das Stechtiefenreferenzelement 8 seine definierte stationäre Position erreicht.

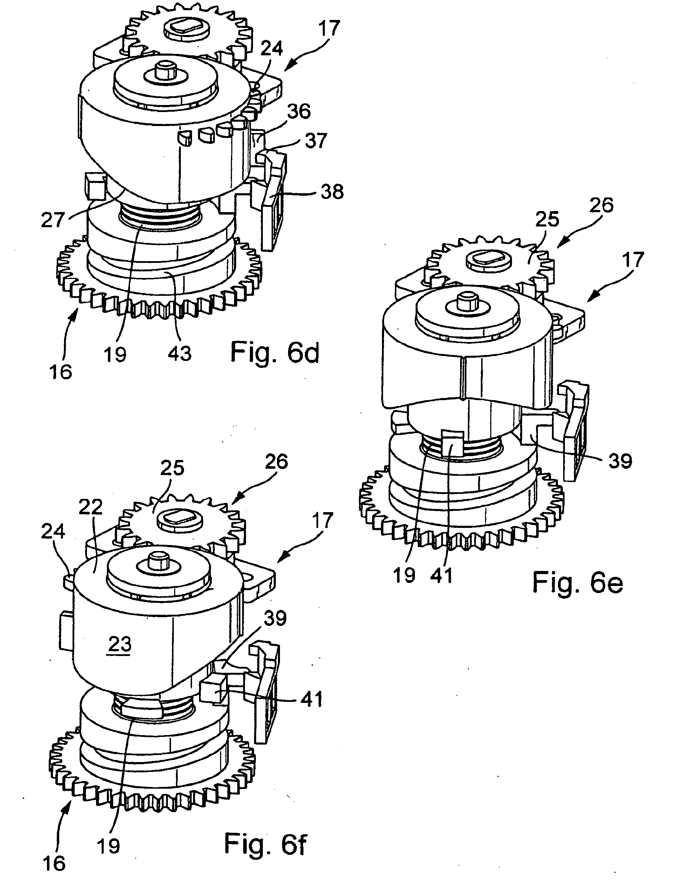

Durch weiteres Drehen des Spannrotors 16 entgegen des Uhrzeigersinns wird die Antriebsfeder 19 vollständig gespannt. Bei Erreichen der in Figur 6c gezeigten 180°-Stellung drückt ein Auslösenocken 39 des Spannrotors 16 gegen einen Auslösehaken 40 der beweglichen Verrastung 38, die dadurch verschwenkt wird. Der Antriebsrotor 17 wird freigegeben und die Antriebsfeder 19 entspannt sich schlagartig. Der Stich wird ausgelöst und die Stechbewegung des Nadelelements 3 beginnt (Figur 6d). Der Spannrotor 16 verbleibt in seiner Stellung, während der Antriebsrotor 17 sehr schnell gegen den Uhrzeigersinn verdreht wird.

Die Drehbewegung des Antriebsrotors 17 wird auf den von der Lanzettenkulisse 27 bewegten Lanzettenhebel 29 so übertragen, dass das Nadelelement 3 durch Entspannen der Antriebsfeder 19 angetrieben wird. Entsprechend der Lanzettenkulisse 27 und der Stichkulisse 43 wird nun das Nadelelement 3 in einer schnellen Bewegung in Einstichrichtung bis zum Erreichen des Umkehrpunkts geführt und anschließend bis zur Reststichtiefe R zurückgezogen. Der Umkehrpunkt der Stechbewegung wird etwa bei einer Drehung des Antriebsrotors 17 bis 90°erreicht.

Zwischen der 90°-Stellung des Antriebsrotors 17 (Figur 6e) und der 180°-Stellung (Figur 6f) greift die Verzahnung 24 des Antriebsrotors 17 in das Zahnrad 25 des Dämpfers 26, wodurch die Bewegungsgeschwindigkeit des Antriebsrotors 17 so abgegebremst wird, dass der Stopp der Drehbewegung des Antriebsrotors 17 gegenüber dem Spannrotor 16 gedämpft erfolgt. Das Nadelelement 3 hat nun die Reststichtiefe R erreicht, die vorzugsweise etwa 0,5 mm beträgt.

Bei der Drehposition des Antriebsrotors 17 von 180° (Figur 6f) trifft ein Nocken 41 des Antriebsrotors 17 (bis z.B. an einen inneren Nockenring des inneren Verstellteils 22) auf den Auslösenocken 39 des Spannrotors 16. Hierdurch werden der Spannrotor 16 und der Antriebsrotor 17 miteinander gekoppelt und durch die Vorspannung der Antriebsfeder 19 gemeinsam bewegt.

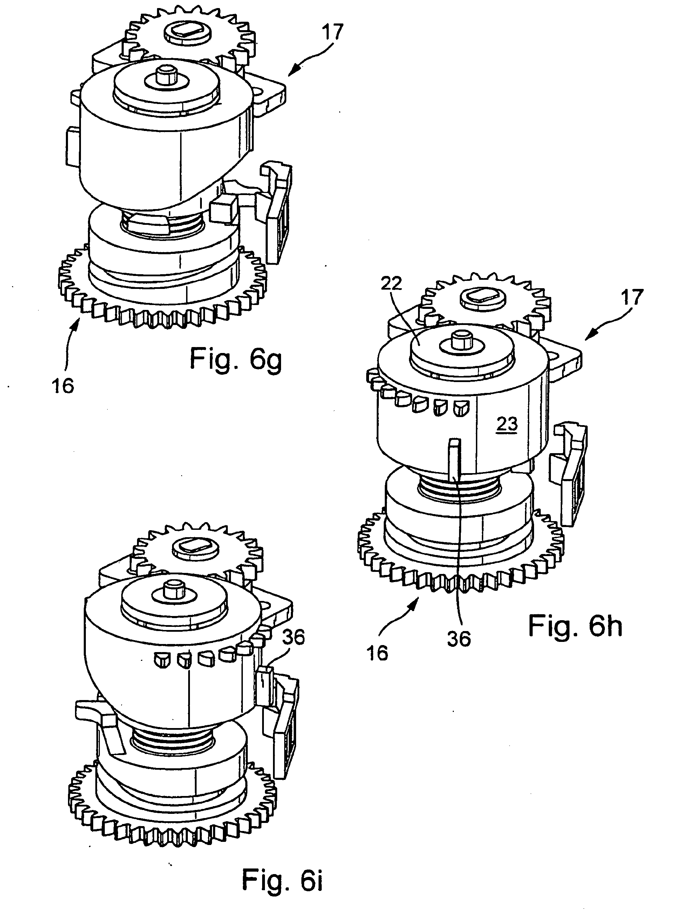

Auf der gemeinsamen Bewegung der beiden Rotoren zwischen 180° und ca. 270° bleibt die Relativposition zwischen dem Nadelelement 3 und dem Stechtiefenreferenzelement 8 konstant. Die gemeinsame Bewegung der Stecheinheit ist durch die parallel verlaufenden Kurven A und D in Figur 7a,b gekennzeichnet. Dieser Bereich ist die Sammelphase, in der die Nadel in der Reststichtiefe R verbleibt und gleichzeitig synchron mit dem Stechtiefenreferenzelement 8 zurückgezogen wird, wie es auch in Figur 2e dargestellt ist. Die Stellung der Rotoren während des Sammelvorgangs ist in Figur 6g dargestellt.

Figur 6h zeigt dann die Drehposition von ca. 270°, bei der der Rückzug des Nadelelements 3 in das Stechtiefenreferenzelement 8 beginnt. Die Kurve D des Nadelelements aus Figur 7 nähert sich der Kurve A des Stechtiefenreferenzelements 8 an und schneidet diese schließlich bei ca. 290°. In diesem Punkt ist das Nadelelement 3 in das Stechtiefenreferenzelement 8 hineingezogen. Nun werden beide in ihre Ruhepositionen zurückgefahren, die bei einer Rotorstellung von 360° (Figur 6i) erreicht wird.

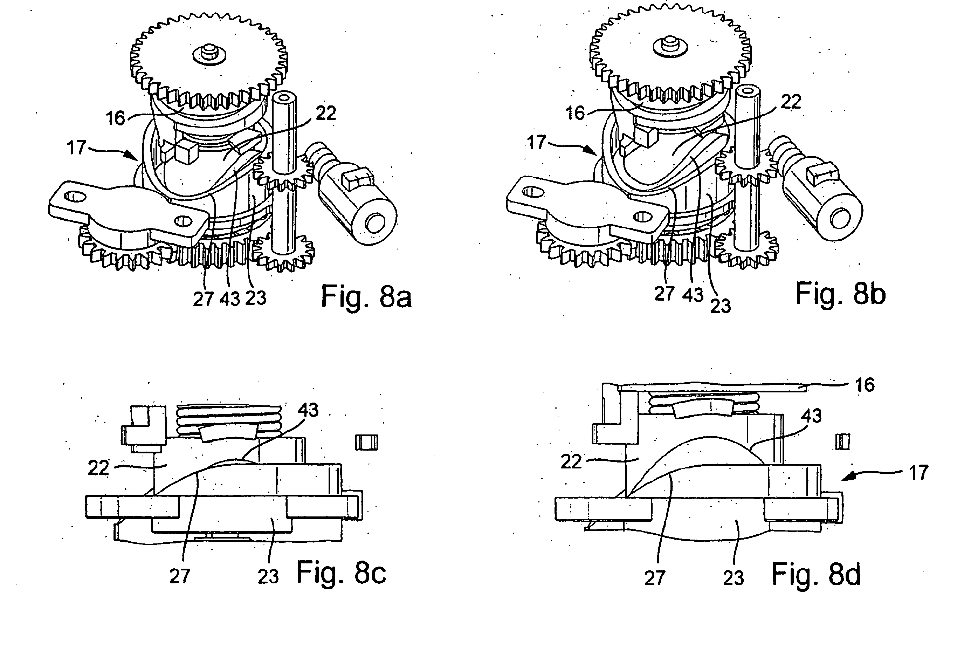

Die beiden Figuren 7a und 7b unterscheiden sich durch eine unterschiedlich eingestellte Stichtiefe. Figur 7a zeigt die minimal eingestellte Stichtiefe, Figur 7b die maximal eingestellte Stichtiefe. Die Kurve C aus Figur 7b ist gegenüber der Kurve C aus Figur 7a verschoben. Diese Verschiebung ergibt sich durch eine Axialverschiebung des Verstellteils 22 gegenüber dem Rotorteil 23, was in den Figuren 8a bis d detailliert gezeigt ist, die Rotoren auf dem Kopf stehend darstellen, damit die Lage im Raum den übrigen Figuren entspricht. Die minimale Stichtiefe beträgt etwa 0,75 mm, die maximale Stichtiefe etwa 2,3 mm.

Da zuerst der Spannrotor 16 bewegt wird bis er eine Drehposition von 180° erreicht hat, wird das Stechtiefenreferenzelement 8 während der Bewegung um 3 mm angehoben. Gleichzeitig wird auch das Nadelelement um diesen Hub von 3 mm angehoben.

Die resultierende Bewegungskurve des Nadelelements 3 (Kurve D) entspricht nach Auslösen des Stichs, also ab dem Moment, in dem sich der Antriebsrotor 17 bewegt, zuerst der Kurve B bis zu einem späteren Drehwinkel die axial verstellbare Kurve C die Führung übernimmt und den Verlauf der Kurve D bestimmt. Ab diesem Drehwinkel folgt der Steuerreiter 33 der Stichkulisse 43 des Verstellteils 22. Ab dem Punkt, ab dem die Kurve C wieder hinter der Kurve B zurückbleibt, führt die Kurve B wieder, das heißt, die Lanzettenkulisse 27 übernimmt wieder die Führung des Steuerreiters 33.

Bei minimaler Stechtiefeneinstellung (Figur 7a) liegen diese Übergabepunkte zwischen den Kurven B und C bei ca. 70° Drehwinkel des Antriebsrotors 17 (von Kurve B nach Kurve C) und bei ca. 105° Drehwinkel (Kurve C nach Kurve B). Bei maximaler Stichtiefe (Figur 7b) führt die Kurve C schon ab 0° Drehwinkel und übergibt die Führung erst bei ca. 120° an die Kurve B.

Bei Erreichen der 180°-Stellung des Antriebsrotors 17 werden der Antriebsrotor 17 und der Spannrotor 16 miteinander gekoppelt. Beide Rotoren bewegen sich parallel. Der Steuerreiter 35 wird nun in der Referenzkulisse 21 geführt, wodurch der Lagerbock 32 nach unten bewegt wird. Somit wird auch das freie Ende des Lanzettenhebels 29, in dem das Kopplungselement 34 angeordnet ist, nach unten geführt. Durch diese Überlagerung der Hebelbewegungen ist es möglich, das Nadelelement 3 synchron und parallel zu dem Stechtiefenreferenzelement 8 zu bewegen, so dass deren relative Position, die der Reststichtiefe entspricht, konstant bleibt. Dies wird durch den Parallelverlauf der Kurven A und D in diesem Drehbereich verdeutlicht.

Ab einer Drehbewegung von 270° beeinflusst die Lanzettenkulisse 27 den Steuerreiter 33 derart, dass das Nadelelement 3 nun wieder der Steuerkurve B parallel folgt. Das Nadelelement 3 wird also gegenüber dem Stechtiefenreferenzelement 8 zurückbewegt, bis es in das Stechtiefenreferenzelement 8 hineingezogen wird.

Die Figuren 8a und b zeigen die um 180 ° gedrehte Antriebseinheit 15 (von unten) in unterschiedlichen Stellungen des Verstellteils 22 in Bezug auf das Rotorteil 23 des Antriebsrotors 17. Figur 8a zeigt die Einstellung der minimalen Stichtiefe. Das Verstellteil 22 ist gegenüber dem Rotorteil 23 axial nach unten, also vom Spannrotor 16 weg, verschoben.

In Figur 8c, die eine Seitenansicht des Antriebs bei Einstellung der minimalen Stichtiefe darstellt, sind die Lanzettenkulisse 27 sowie die Stichkulisse 43 gezeigt, die für die Bewegung des Nadelelements verantwortlich sind. Die Kulisse 43 ragt nur wenig über die Lanzettenkulisse hervor, so dass die Nadel bzw. Lanzette ebenfalls nur wenig in die Haut eindringt.

Die Figuren 8b und d zeigen die Antriebseinheit 15 in der Einstellung der maximalen Stichtiefe. Das Verstellteil 22 ist gegenüber dem Rotorteil 23 axial in Richtung Spannrotor 16 verschoben. Der Steuerreiter zur Steuerung der Bewegung der Lanzette wird von der Kulisse 43 geführt, die gegenüber der Lanzettenkulisse 27 deutlich abgehoben ist. Hieraus ergibt sich eine große Stichtiefe. Die Kulisse 43 bestimmt die Bewegung des Nadelelements 3 während der gesamten Vortriebsphase und des schnell ablaufenden Teils sder Rückführungsphase.

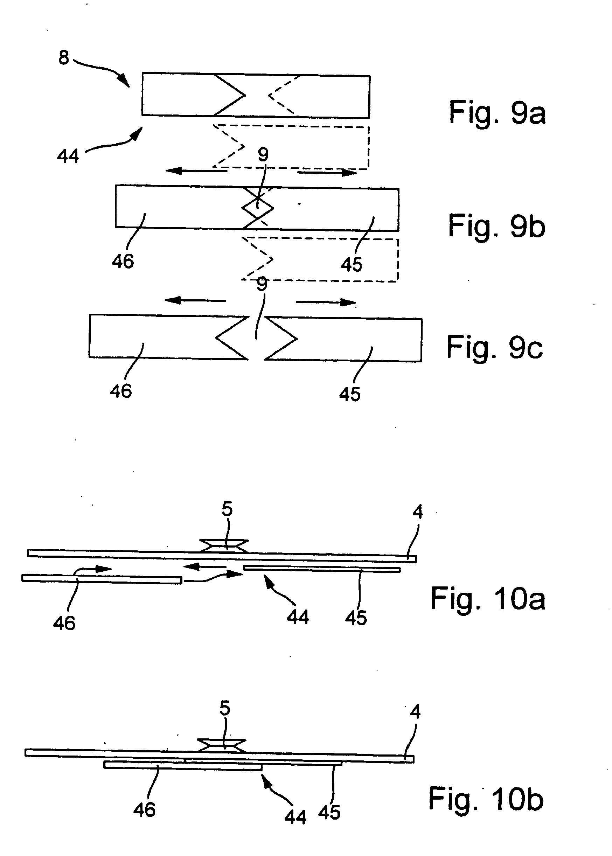

Figur 9 zeigt ein Stechtiefenreferenzelement 8, das mit einem zweiteiligen Verschluss 44 mit einer Elementöffnung 9 ausgebildet ist. Durch die Elementöffnung 9 des ebenfalls als bevorzugte Ausführungsform angesehenen Stechtiefenreferenzelements 8 tritt die Spitze 3c des Nadelelements 3 in der Vortriebsphase der Stechbewegung hindurch. Die Elementöffnung 9 ist vorteilhafterweise in ihrer Größe veränderbar. Sie kann auch soweit reduziert werden, dass das Stechtiefenreferenzelement 8 geschlossen wird.

Die Elementöffnung 9 des Stechtiefenreferenzelements 8 ist dabei so dimensioniert, dass die an der Referenz-Hautkontaktfläche 11 anliegende Haut gestrafft wird. Der minimale Abstand zwischen der Referenz-Hautkontaktfläche 11 und dem Nadelelement 3 beträgt allgemein, wie es auch schon weiter oben für ein Stechtiefenreferenzelement ohne Elementöffnung beschrieben wurde, höchstens 1,5 mm, bevorzugt höchstens 1 mm, besonders bevorzugt höchstens 0,5 mm. Wird diese kleinste Entfernung zwischen der Referenz-Hautkontaktfläche 11 und dem Nadelelement 3 eingehalten, so ergibt sich bei Stechtiefenreferenzelementen 8 mit einer Elementöffnung 9 ein entsprechender Öffnungsquerschnitt. Der Querschnitt der Elementöffnung 9 ist so bemessen, dass bei einer kreisförmigen Öffnung der Querschnitt einem Durchmesser von höchstens 3 mm entspricht, besonders bevorzugt von höchstens 2 mm. Nicht kreisförmige Elementöffnungen 9 haben einen entsprechenden Querschnitt. Mögliche ebenfalls bevorzugte Querschnittsformen der Elementöffnung sind vier-oder mehreckige Querschnitte, besonders bevorzugt sind Öffnungen mit rautenförmigem Querschnitt.

Vorzugsweise besteht das Stechtiefenreferenzelement 8 aus einer Mehrzahl von Bauteilen. Die Referenz-Hautkontaktfläche 11 ist bevorzugt an der Mehrzahl von Bauteilen ausgebildet, die zur Veränderung der Größe der Elementöffnung 9 relativ zueinander beweglich sind. Die Bauteile des Stechtiefenreferenzelements werden derart bewegt, dass sie eine Bewegungskomponente senkrecht zur Einstichrichtung der Stechbewegung aufweisen. Ein Beispiel für ein derartiges Stechtiefenreferenzelement 8 ist beispielsweise eine Lochblende mit einem veränderbaren Lochquerschnitt.

Der zweiteilige Verschluss 44 in den Figuren 9a bis c weist die beiden Verschlusselemente 45 und 46 auf. Die Verschlusselemente 45 und 46 können gegeneinander so verschoben werden, dass sich der Verschluss 44 komplett verschließen lässt (Figur 9a). Das komplett verschließbare Stechtiefenreferenzelement 8 kann dann das gesamte Stechsystem 1 verschließen, wenn das Stechtiefenreferenzelement 8 schließend und/oder dichtend an der Gehäuseöffnung 5 des Stechgeräts 2 anliegt.

Figur 9b zeigt den Verschluss 44 des Stechtiefenreferenzelements 8 mit einer kleinen Elementöffnung 9, durch die das Nadelelement 3 heraustreten kann. Figur 9c zeigt den komplett geöffneten Verschluss 44, bei dem die beiden Verschlusselemente 45 und 46 voneinander beabstandet sind. Es wird eine Elementöffnung 9 mit einem sehr großen Querschnitt gebildet, so dass das Blut aus der Wunde nach dem Einstich gesammelt werden kann. Insbesondere kann eine Analyseeinheit zur Aufnahme des Blutstropfen in die Elementöffnung 9 gebracht werden und das austretende Blut auf einen Probenträger gelangen.

In Figur 10 ist der Verschluss 44 in einer Seitenansicht dargestellt. Figur 10a zeigt den geöffneten Verschluss, bei dem die beiden Verschlusselemente 45,46 voneinander beabstandet sind. Beim Verschließen des Verschlusses 44 werden die Verschlusselemente 45,46 aufeinander zubewegt, wobei das Verschlusselement 46 gegen das Verschlusselement 45 und dieses gegen das Gehäuse 4 drückt, so dass die Gehäuseöffnung 5 verschlossen wird.

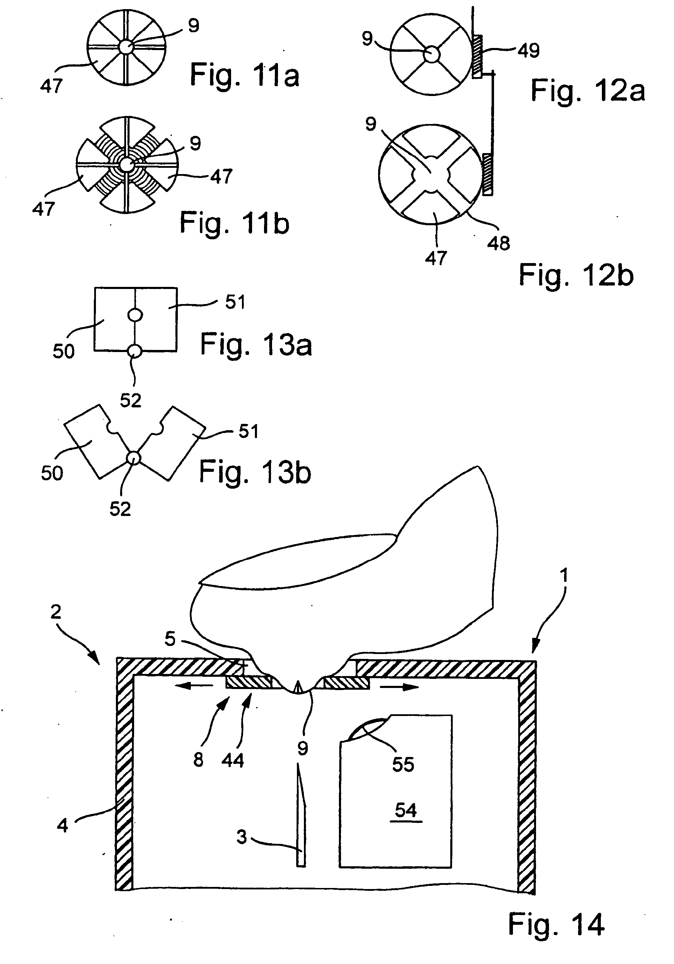

In den Figuren 11a,b und 12a,b sind weitere Ausführungsformen eines als Verschluss 44 ausgebildeten Stechtiefenreferenzelement 8 dargestellt. Der mehrteilige Verschluss weist eine Elementöffnung 9 auf. In der Ausführungsform nach Figur 11b werden die Verschlussteile 47 durch Drehen des Verschlusses 44 radial nach außen bewegt, um die Elementöffnung 9 zu vergrößern.

In der Ausführungsform nach Figur 12a,b werden die beispielsweise federbelasteten Verschlussteile 47 von einer Schelle 48 zusammengehalten, die über eine Spindel 49 verstellt werden kann. Wenn die Schelle 48 aufgeweitet wird, werden die Verschlussteile 47 radial nach außen bewegt und die Elementöffnung 9 vergrößert, Figur 12b.

Die Figuren 13a,b zeigen ein zweiteilig ausgebildetes Stechtiefenreferenzelement 8, dessen zwei Verschlusselemente 50,51 um ein Scharnier 52 gegeneinander verschwenkt werden können. Durch dieses Aufklappen der Elemente 50,51 wird die Elementöffnung 9 vergrößert, so dass nach der Stechbewegung der Lanzette bzw. des Nadelelements eine große Öffnungsfläche freigegeben wird, in die auch eine Analyseeinheit bewegt werden kann, um nach dem Einstich aus der Wunde austretendes Blut aufzunehmen.

Die in den Figuren 9 bis 13 gezeigten Stechtiefenreferenzelemente 8 mit veränderlichen Elementöffnungen 9 eignen sich besonders für Blutgewinnungsverfahren, bei denen die Probe nicht vom Nadelelement aufgenommen wird, sondern sich an der Hautoberfläche sammelt, um von einem Analyseelement direkt aufgenommen zu werden. Solche Blutgewinnungsverfahren werden mit einem Stechsystems 1 durchgeführt, das neben einer Stecheinheit auch eine Analyseeinheit 54 aufweist. Die Analyseeinheit 54 hat wenigstens ein Analyseelement 55 zur Aufnahme der Flüssigkeit, die bevorzugt das aus dem Finger 13 austretende Blut ist. Ein in dem Blut enthaltener Anlayt, insbesondere die Glucose, wird in der Analyseeinheit 54 bestimmt.

Derartige Stechgeräte mit einer integrierten Analyseeinheit sind auch aus der WO 2005/006985 A2 bekannt. Einzelheiten der vorzugsweise kassettenartigen Analyseeinheit mit einem bandförmigen Analyseelement sind in der genannten Druckschrift detailliert beschrieben und werden deshalb an dieser Stelle nicht explizit ausgeführt. Der Inhalt der WO 2005/006985 A2 wird durch Referenzierung in diese Anmeldung integriert.

Figur 14 zeigt beispielhaft ein Stechsystem 1 mit einer Analyseeinheit 54, die in einem Stechgerät 2 angeordnet ist. Hinter der Gehäuseöffnung 5 des Stechgeräts 2 ist ein mehrteiliges Stechtiefenreferenzelement 8 angeordnet, das in Form des aus Figur 9 bekannten zweiteiligen Verschlusses 44 ausgebildet ist.

Der Blutgewinnungsvorgang läuft mit dem Stechgerät 2 deutlich anders ab als er in den Figuren 2, 6 und 7 beschrieben ist. Das Nadelelement 3 bewegt sich während seiner Stechbewegung in der Vortriebsphase, wie auch in der Rückführungsphase, schnell. Es sticht also schnell in den Finger 13 ein und zieht sich ebenso schnell vollständig wieder aus ihm zurück. Während des Stechvorgangs ist das Stechtiefenreferenzelement 8 weitgehend geschlossen, so dass nur eine Elementöffnung 9 entsteht, deren Querschnitt einem runden Querschnitt mit ca. 2 mm Durchmesser entspricht.

Nachdem das Nadelelement 3 aus der Haut 14 des Fingers 13 zurückgezogen worden ist, werden die Verschlusselemente 45 und 46 jeweils in Pfeilrichtung seitwärts bewegt, so dass die Elementöffnung 9 vergrößert wird. Vorzugsweise wird die Elementöffnung 9 dann soweit aufgeweitet, dass sie wenigstens der Gehäuseöffnung 5 entspricht.

Die Auseinanderbewegung der Verschlusselemente 45 und 46 des Stechtiefenreferenzelements 8 hat zur Folge, dass sich der Finger 13 in die Gehäuseöffnung 5 weiter hineinwölbt. Gleichzeitig wird der von dem Stechtiefenreferenzelement 8 gegen den Finger 13 ausgeübte Gegendruck reduziert bzw. aufgehoben. Der Blutaustritt aus der Wunde wird nicht mehr verhindert. Das in der Hautwölbung des Fingers gesammelte Blut kann aus der Einstichwunde heraustreten. Es wird somit eine expressionsfördernde Wirkung durch die Gehäuse-Hautkontaktfläche 7 erzielt. Das austretende Blut steht zur Aufnahme durch das Analyseelement 55 bereit.

Nachdem die Verschlusselemente 45 und 46 in ihre maximale Öffnungsposition bewegt worden sind, wird die Analyseeinheit 54 derart in Richtung Gehäuseöffnung 5 bewegt, dass das aus der Einstichwunde austretende Blut von dem Analyseelement 55 aufgenommen werden kann.

Bevorzugt ist eine Ausführungsform des Stechgeräts 2, bei dem die beiden Verschlusselemente 45 und 46 seitwärts in Bezug auf die Einstichrichtung bewegt werden, wobei die Seitswärtsbewegung dadurch definiert ist, dass sie eine Bewegungskomponente senkrecht zur Einstichrichtung aufweist. Dies schließt eine Bewegung senkrecht zur Einstichrichtung mit ein. Die Analyseeinheit 54 wird bevorzugt so bewegt, dass sie eine Bewegungskomponente parallel zur Einstichrichtung des Nadelelements 3 aufweist. Darüber hinaus kann ihre Bewegung auch senkrecht zur Einstichrichtung orientierte Bewegungskomponenten haben. Im einfachsten Fall wird die Analyseeinheit 54 parallel zur Einstichrichtung des Nadelelements 3 bewegt. Auf dem Bewegungsweg der Analyseeinheit wird sie von einer Ausgangsposition in eine Position hinter der Gehäuseöffnung 5 bewegt, wobei die Analyseeinheit dann auch gegen den sich in die Gehäuseöffnung 5 einwirkenden Finger 13 drücken kann.

In einer alternativen Ausführungsform ist das Stechtiefenreferenzelement 8 auch bei einem Stechgerät 2 mit integrierter Analyseeinheit 54 einstückig ausgebildet. In diesem Fall wird das Stechtiefenreferenzelement 8 bevorzugt ebenfalls seitwärts von der Gehäuseöffnung 5 wegbewegt, wobei die Bewegung eine senkrecht zur Einstichrichtung orientierte Bewegungskomponente aufweist. Die Bewegung kann also auch schräg nach hinten erfolgen.

## Patentansprüche

1. Stechsystem zur Entnahme einer Körperflüssigkeit aus der Haut eines Menschen oder Tieres, umfassend
ein Nadelelement (3) zum Einstechen in die Haut,
ein Stechgerät (2), das einen Stechantrieb (15a) einschließt, durch den eine Stechbewegung des mittels eines Kopplungsmechanismus (28) mit dem Stechantrieb gekoppelten Nadelelements (3) in Einstichrichtung angetrieben wird,
wobei
- das Stechgerät (2) ein Gehäuse (4) mit einer Gehäuseöffnung (5) an seinem in Einstichrichtung vorderen Ende aufweist und die Gehäuseöffnung (5) von einer Gehäuse-Hautkontaktfläche (7) umgeben ist, die bei der Benutzung des Stechgerätes (2) gegen die Haut gedrückt wird;
- das Nadelelement (3) in einer Vortriebsphase der Stechbewegung auf einem vorbestimmten Einstichweg in einer Einstichrichtung bewegt wird, bis seine Spitze (3c) in die Haut eindringt, um eine Wunde zu erzeugen und in einer Rückführungsphase der Stechbewegung nach Erreichen eines der Stichtiefe (S) in die Haut entsprechenden Umkehrpunktes wieder zurückgezogen wird;
- zur Verbesserung der Reproduzierbarkeit der Stichtiefe (S) ein Stechtiefenreferenzelement (8) mit einer Referenz-Hautkontaktfläche (11) vorgesehen ist, das so ausgebildet und angeordnet ist, dass die Referenz-Hautkontaktfläche (11) am Umkehrpunkt der Stechbewegung mit der Haut in Kontakt steht und der vorgegebene Wert der Stichtiefe (S) durch den Abstand in Einstichrichtung bestimmt wird, der an dem Umkehrpunkt der Stechbewegung zwischen der Referenz-Hautkontaktfläche (11) und der Position der Spitze (3c) des Nadelelements (3) besteht, wobei zu dem Zeitpunkt, zu dem die Spitze (3c) des Nadelelements (3) in der Vortriebsphase der Stechbewegung die Referenz-Hautkontaktfläche (11) passiert und in eine mit dieser in Kontakt stehenden Hautoberfläche eindringt, das Stechtiefenreferenzelement (8) mit der Referenz-Hautkontaktfläche (11) in einer stationären definierten Position in Bezug auf den Umkehrpunkt der Stechbewegung ist;
- das Stechtiefenreferenzelement (8) derartig ausgebildet und angeordnet ist, dass die Haut beim Andrücken an die Referenz-Hautkontaktfläche (11) hinsichtlich einer beim Einstich in die Haut auftreffenden Hautdeformation stabilisiert wird;
- das Nadelelement (3) einen Kapillarkanal (3b) aufweist, durch den eine Körperflüssigkeit aus der Haut transportiert werden kann;
- das Nadelelement nach dem Umkehrpunkt der Stechbewegung entgegen der Einstichrichtung auf eine Reststichtiefe zurückgezogen wird; und
- danach während einer Sammelphase zur Aufnahme der Körperflüssigkeit eine gemeinsame Bewegung des Stechtiefenreferenzelementes (8) und des Nadelelementes (3) entgegen der Einstichrichtung stattfindet, bevor die Spitze (3c) des Nadelementes (3) vollständig aus der Haut herausgezogen ist.

2. Stechsystem nach Anspruch 1, **dadurch gekennzeichnet, dass** sich das Stechtiefenreferenzelement (8) mit der Referenz-Hautkontaktfläche (11) vor dem Andrücken des Stechgerätes (2) an die Haut in einer auch bezüglich der Gehäuse-Hautkontaktfläche (7) definierten Startposition befindet.

3. Stechsystem nach Anspruch 2, **dadurch gekennzeichnet, dass** die definierte Startposition des Stechtiefenreferenzelements (8) mit der Referenz-Hautkontaktfläche (11), bezogen auf die Gehäuse-Hautkontaktfläche (7) durch Kontakt eines Stechtiefenreferenzelementanschlags (10) und eines mit diesem korrespondierenden Gehäuseanschlags (12) definiert ist.

4. Stechsystem nach einem der vorhergehenden Ansprüche, **dadurch gekennzeichnet, dass** das Stechtiefenreferenzelement (8) eine Elementöffnung (9) aufweist, durch die die Spitze (3c) des Nadelelements (3) in der Vortriebsphase der Stechbewegung hindurchtritt und die so ausgebildet ist, dass der kleinste senkrecht zur Einstichrichtung gemessene Abstand zwischen dem Nadelelement (3) und der Referenz-Hautkontaktfläche (11) des Stechtiefenreferenzelements (8) höchstens 2 mm, vorzugsweise höchstens 1 mm und besonders bevorzugt höchstens 0,5 mm beträgt.

5. Stechsystem nach einem der vorhergehenden Ansprüche, **dadurch gekennzeichnet, dass** es eine Analyseeinheit (54) aufweist, die wenigstens ein Analyseelement (55) zur Aufnahme einer Flüssigkeit und zur Bestimmung eines in der Flüssigkeit enthaltenen Analyten umfasst.

6. Verfahren zur Entnahme einer Körperflüssigkeit aus der Haut eines Menschen oder Tieres, mittels
eines Nadelelementes (3) zum Einstechen in die Haut und
eines Stechgerätes (2), das
- ein Gehäuse (4) mit einer von einer Gehäuse-Hautkontaktfläche (7) umgebenen Gehäuseöffnung (5) an seinem in Einstichrichtung vorderen Ende aufweist,
- einen Stechantrieb (15a) einschließt, durch den eine Stechbewegung eines mittels eines Kopplungsmechanismus (28) mit dem Stechantrieb gekoppelten Nadelelements (3) in Einstichrichtung angetrieben wird, und
- zur Verbesserung der Reproduzierbarkeit der Stichtiefe (S) ein Stechtiefenreferenzelement (8) mit einer Referenz-Hautkontaktfläche (11) aufweist, das so ausgebildet und angeordnet ist, dass die Referenz-Hautkontaktfläche (11) am Umkehrpunkt der Stechbewegung mit der Haut in Kontakt steht und der vorgegebene Wert der Stichtiefe (S) durch den Abstand in Einstichrichtung bestimmt wird, der an dem Umkehrpunkt der Stechbewegung zwischen der Referenz-Hautkontaktfläche (11) und der Position der Spitze (3c) des Nadelelements (3) besteht,
wobei
die Hautkontaktfläche des Stechgerätes (2) bei der Benutzung des Stechgerätes (2) gegen die Haut gedrückt wird;
das Nadelelement (3) in einer Vortriebsphase der Stechbewegung auf einem vorbestimmten Einstichweg in einer Einstichrichtung bewegt wird, bis seine Spitze (3c) in die Haut eindringt, um eine Wunde zu erzeugen und in einer Rückführungsphase der Stechbewegung nach Erreichen eines der Stichtiefe (S) in die Haut entsprechenden Umkehrpunktes wieder zurückgezogen wird;
zu dem Zeitpunkt, zu dem die Spitze (3c) des Nadelelements (3) in der Vortriebsphase der Stechbewegung die Referenz-Hautkontaktfläche (11) passiert und in eine mit dieser in Kontakt stehende Hautoberfläche eindringt, das Stechtiefenreferenzelement (8) mit der Referenz-Hautkontaktfläche (11) in einer stationären definierten Position in Bezug auf den Umkehrpunkt der Stechbewegung ist;
das Stechtiefenreferenzelement (8) derartig ausgebildet und angeordnet ist, dass die Haut beim Andrücken an die Referenz-Hautkontaktfläche (11) hinsichtlich einer beim Einstich in die Haut auftreffenden Hautdeformation stabilisiert wird;
das Nadelelement (3) einen Kapillarkanal (3b) aufweist, durch den eine Körperflüssigkeit aus der Haut transportiert werden kann;
das Nadelelement nach dem Umkehrpunkt der Stechbewegung entgegen der Einstichrichtung auf eine Reststichtiefe zurückgezogen wird; und
danach während einer Sammelphase zur Aufnahme der Körperflüssigkeit eine gemeinsame Bewegung des Stechtiefenreferenzelementes (8) und des Nadelelementes (3) entgegen der Einstichrichtung stattfindet, bevor die Spitze (3c) des Nadelementes (3) vollständig aus der Haut herausgezogen ist.

7. Verfahren nach Anspruch 6, **dadurch gekennzeichnet, dass** sich das Stechtiefenreferenzelement (8) mit der Referenz-Hautkontaktfläche (11) vor dem Andrücken des Stechgerätes (2) an die Haut in einer auch bezüglich der Gehäuse-Hautkontaktfläche (7) definierten Startposition befindet.

8. Stechsystem nach einem der Ansprüche 1 bis 5 oder Verfahren nach einem der Ansprüche 6 oder 7, **dadurch gekennzeichnet, dass** das Stechtiefenreferenzelement (8) und das Nadelelement (3) während eines Teils der Rückführungsphase derart synchron bewegt werden, dass die relative Position zwischen der Spitze (3c) des Nadelelements (3) und der Referenz-Hautkontaktfläche (11) des Stechtiefenreferenzelements (8) unverändert bleibt.

9. Stechsystem nach einem der Ansprüche 1 bis 5 oder Verfahren nach einem der Ansprüche 6 oder 7, **dadurch gekennzeichnet, dass** die Rückführungsphase der Stechbewegung zwei Teilabschnitte einschließt, wobei die Bewegung des Nadelelements (3) in dem ersten Teilabschnitt schneller als in dem zweiten Teilabschnitt ist und während der schnellen Bewegung des ersten Teilabschnitts der Rückführungsphase die Referenz-Hautkontaktfläche (11) in der stationären Position verbleibt.

10. Stechsystem nach einem der Ansprüche 1 bis 5 oder Verfahren nach einem der Ansprüche 6 oder 7, **dadurch gekennzeichnet, dass** das Stechtiefenreferenzelement (8), bevor die Spitze (3c) des Nadelelements (3) vollständig aus der Haut herausgezogen ist, höchstens so weit aus seiner stationären Position relativ zu dem Gehäuse (4) entgegen der Einstichrichtung bewegt wird, dass die Referenz-Hautkontaktfläche (11) mit der Haut in Kontakt bleibt.

## Claims

1. Lancing system for extracting a body fluid from the skin of a human or animal, comprising
a needle element (3) for lancing the skin,
a lancing device (2), including a lancet drive (15a) by which a lancing movement of the needle element (3) is driven in a lancing direction, the needle element (3) being coupled with the lancet drive by means of a coupling mechanism (28),
wherein
- the lancing device (2) comprises a housing (4) with a housing opening (5) at its in lancing direction front end and the housing opening (5) is surrounded by a housing skin contact surface (7) which is pressed against the skin during use of the lancing device (2);
- the needle element (3) is, during a forward phase of the lancing movement, moved on a predetermined lancing path in a lancing direction until its tip (3c) enters the skin in order to create a wound and is again retracted in a return phase of the lancing movement after having reached a reversal point, the reversal point corresponding to the lancing depth (S) in the skin;
- a lancing depth reference element (8) is provided for improving the reproducibility of the lancing depth (S) the lancing depth reference element comprising a reference skin contact surface (11) and being adapted and arranged in such a manner that the reference skin contact surface (11) is, at the reversal point of the lancing movement, in contact with the skin and the predetermined value of the lancing depth (S) is determined by the distance in lancing direction that exists at the reversal point of the lancing movement between the reference skin contact surface (11) and the position of the tip (3c) of the needle element (3), wherein at the time at which the tip (3c) of the needle element (3), in the forward phase of the lancing movement, passes the reference skin contact surface (11) and enters a skin surface which is in contact with said reference skin contact surface, the lancing depth reference element (8) with the reference skin contact surface (11) is in a stationary defined position relative to the reversal point of the lancing movement;
- the lancing depth reference element (8) is adapted and arranged in such a manner that the skin, on pressing against the reference skin contact surface (11), is stabilized with respect to a skin deformation occurring when lancing the skin;
- the needle element (3) comprises a capillary channel (3b) through which a body fluid can be transported out of the skin;
- after the reversal point of the lancing movement the needle element is retracted to a residual depth; and
- thereafter, during a collecting phase for taking up of the body fluid, a common movement of the lancing depth reference element (8) and the needle element (3), opposite to the lancing direction, takes place before the tip (3c) of the needle element (3) is completely withdrawn from the skin.

2. Lancing system according to Claim 1, **characterized in that** before the lancing device (2) is pressed to the skin, the lancing depth reference element (8) with the skin contact surface (11) is in a defined starting position also relative to the housing skin contact surface (7).

3. Lancing system according to Claim 2, **characterized in that** the defined starting position of the lancing depth reference element (8) with the reference skin contact surface (11), relative to the housing skin contact surface (7), is defined by the contact of a stop (10) of the lancing depth reference element with a corresponding housing stop (12).

4. Lancing system according to any one of the preceding claims, **characterized in that** the lancing depth reference element (8) comprises an element opening (9) through which the tip (3c) of the needle element (3) extends in the forward phase of the lancing movement and the element opening (9) is shaped such that the smallest distance between the needle element (3) and the reference skin contact surface (11) of the lancing depth reference element (8) measured vertically to the lancing direction, is at most 2 mm, preferentially at most 1 mm and particularly preferably at most 0.5 mm.

5. The lancing system according to any one of the preceding claims, **characterized in that** it comprises an analysis unit (54) which comprises at least one analysis element (55) for taking up a fluid and for determining an analyte contained in the fluid.

6. Method for extracting a body fluid from the skin of a human or animal, using
a needle element (3) for lancing the skin and
a lancing device (2), which
- comprises a housing (4) with a housing opening (5) at its front end in lancing direction, and the housing opening (5) being surrounded by a housing skin contact surface (7),
- comprises a lancet drive (15a) by which a lancing movement of the needle element (3) is driven in a lancing direction, the needle element (3) being coupled with the lancet drive by means of a coupling mechanism (28), and
- comprises a lancing depth reference element (8) for improving the reproducibility of the lancing depth (S), the lancing depth reference element comprising a reference skin contact surface (11) and being adapted and arranged in such a manner that the reference skin contact surface (11) is, at the reversal point of the lancing movement, in contact with the skin and the predetermined value of the lancing depth (S) is determined by the distance in lancing direction that exists at the reversal point of the lancing movement between the reference skin contact surface (11) and the position of the tip (3c) of the needle element (3),
wherein
the skin contact surface of the lancing device (2) is pressed against the skin during use of the lancing device (2);
the needle element (3) is, during a forward phase of the lancing movement, moved on a predetermined lancing path in a lancing direction until its tip (3c) enters the skin in order to create a wound and is again retracted in a return phase of the lancing movement after having reached a reversal point, the reversal point corresponding to the lancing depth (S) in the skin;
at the time at which the tip (3c) of the needle element (3), in the forward phase of the lancing movement, passes the reference skin contact surface (11) and enters a skin surface which is in contact with said reference skin contact surface, the lancing depth reference element (8) with the reference skin contact surface (11) is in a stationary defined position relative to the reversal point of the lancing movement;
the lancing depth reference element (8) is adapted and arranged in such a manner that the skin, on pressing against the reference skin contact surface (11), is stabilized with respect to a skin deformation occurring when lancing the skin;
the needle element (3) comprises a capillary channel (3b) through which a body fluid can be transported out of the skin;
after the reversal point of the lancing movement the needle element is retracted to a residual depth; and
thereafter, during a collecting phase for taking up of the body fluid, a common movement of the lancing depth reference element (8) and the needle element (3), opposite to the lancing direction, takes place before the tip (3c) of the needle element (3) is completely withdrawn from the skin.

7. Method according to Claim 6, **characterized in that** before the lancing device (2) is pressed to the skin, the lancing depth reference element (8) with the skin contact surface (11) is in a defined starting position also relative to the housing skin contact surface (7).

8. Lancing system according to any one of Claims 1 to 5 or method according to any one of Claims 6 or 7, **characterized in that** the lancing depth reference element (8) and the needle element (3) are moved in synchronization during a part of the return phase in such a manner that the relative distance between the tip (3c) of the needle element (3) and the reference skin contact surface (11) of the lancing depth reference element (8) remains unchanged.

9. Lancing system according to any one of Claims 1 to 5 or method according to any one of Claims 6 or 7, **characterized in that** the return phase of the lancing movement includes two part sections wherein the movement of the needle element (3) is faster in the first part section than in the second part section and wherein during the rapid movement of the first part section of the return phase the reference skin contact surface (11) remains in the stationary position.

10. Lancing system according to any one of Claims 1 to 5 or method according to any one of Claims 6 or 7, **characterized in that**, before the tip (3c) of the needle element (3) is completely extracted from the skin, the lancing depth reference element (8) is moved from its stationary position relative to the housing (4) and opposite to the lancing direction only so far that the reference skin contact surface (11) remains in contact with the skin.

## Revendications

1. Système de piqûre destiné au prélèvement d'un liquide corporel à travers la peau d'un homme ou d'un animal, comprenant
un élément aiguille (3) pour piquer la peau,
un appareil de piqûre (2) qui comporte un entraînement de piqûre (15a) par lequel un mouvement de piqûre de l'élément aiguille (3) couplé audit entraînement de piqûre au moyen d'un mécanisme de couplage (28) est entraîné dans le sens de la piqûre,
- l'appareil de piqûre (2) présentant un boîtier (4) muni d'une ouverture de boîtier (5) à son extrémité avant dans le sens de la piqûre et ladite ouverture de boîtier (5) étant entourée d'une surface de contact boîtier-peau (7) qui est pressée contre la peau lors de l'utilisation de l'appareil de piqûre (2) ;
- l'élément aiguille (3), durant une phase d'avancement du mouvement de piqûre, étant déplacé sur un trajet de piqûre prédéterminé, dans un sens de piqûre, jusqu'à ce que sa pointe (3c) pénètre dans la peau pour provoquer une blessure et étant de nouveau retiré durant une phase de recul du mouvement de piqûre, après avoir atteint un point d'inversion correspondant à la profondeur de piqûre (S) dans la peau ;
- un élément de référence de profondeur de piqûre (8) doté d'une surface de référence de contact cutané (11) étant prévu pour améliorer la reproductibilité de la profondeur de piqûre (S), lequel élément est conçu et disposé de telle façon que la surface de référence de contact cutané (11) est en contact avec la peau au point d'inversion du mouvement de piqûre et que la valeur prédéfinie de la profondeur de piqûre (S) est déterminée par la distance, dans le sens de la piqûre, qui existe entre la surface de référence de contact cutané (11) et la position de la pointe (3c) de l'élément aiguille (3) au point d'inversion du mouvement de piqûre, sachant qu'au moment où la pointe (3c) de l'élément aiguille (3), durant la phase d'avancement du mouvement de piqûre, passe à travers la surface de référence de contact cutané (11) et pénètre dans une surface cutanée en contact avec celle-ci, l'élément de référence de profondeur de piqûre (8), doté de la surface de référence de contact cutané (11), est dans une position stationnaire définie par rapport au point d'inversion du mouvement de piqûre ;
- l'élément de référence de profondeur de piqûre (8) étant conçu et disposé de telle façon que la peau, lorsqu'elle est pressée contre la surface de référence de contact cutanée (11), est stabilisée en termes de déformation de la peau se produisant lorsque l'on pique la peau ;
- l'élément aiguille (3) présentant un canal capillaire (3b) à travers lequel un liquide corporel peut être transporté hors de la peau ;
- ledit élément aiguille, après le point de conversion du mouvement de piqûre, étant ramené en arrière dans le sens contraire du mouvement de piqûre jusqu'à une profondeur de piqûre résiduelle ; puis
- durant une phase de collecte pour recueillir le liquide corporel, un mouvement conjoint de l'élément de référence de profondeur de piqûre (8) et de l'élément aiguille (3) ayant lieu dans le sens contraire de la piqûre, avant que la pointe (3c) de l'élément aiguille (3) ne soit totalement retirée de la peau.

2. Système de piqûre selon la revendication 1, **caractérisé en ce qu'**avant de presser l'appareil de piqûre (2) contre la peau, l'élément de référence de profondeur de piqûre (8), doté de la surface de référence de contact cutané (11), se trouve dans une position de départ définie, également par rapport à la surface de contact boîtier-peau (7).

3. Système de piqûre selon la revendication 2, **caractérisé en ce que** la position de départ définie de l'élément de référence de profondeur de piqûre (8), doté de la surface de référence de contact cutané (11), par rapport à la surface de contact boîtier-peau (7), est définie par le contact d'une butée (10) d'élément de référence de profondeur de piqûre et d'une butée (12) de boîtier en correspondance avec cette dernière.

4. Système de piqûre selon l'une des revendications précédentes, **caractérisé en ce que** l'élément de référence de profondeur de piqûre (8) présente une ouverture (9) à travers laquelle passe la pointe (3c) de l'élément aiguille (3) durant la phase d'avancement du mouvement de piqûre et qui est conçue de telle façon que la plus petite distance mesurée perpendiculairement au sens de la piqûre, entre l'élément aiguille (3) et la surface de référence de contact cutané (11) de l'élément de référence de profondeur de piqûre (8), est tout au plus de 2 mm, de manière préférée tout au plus de 1 mm et de manière particulièrement préférée tout au plus de 0,5 mm.

5. Système de piqûre selon l'une des revendications précédentes, **caractérisé en ce qu'**il présente une unité d'analyse (54) qui comprend au moins un élément d'analyse (55) destiné à recueillir un liquide et à déterminer un analyte contenu dans le liquide.

6. Procédé de prélèvement d'un liquide corporel à travers la peau d'un homme ou d'un animal, au moyen
d'un élément aiguille (3) pour piquer la peau et
d'un appareil de piqûre (2) qui
- présente un boîtier (4) muni d'une ouverture de boîtier (5) à son extrémité avant dans le sens de la piqûre, entourée d'une surface de référence de contact cutané (7),
- comporte un entraînement de piqûre (15a) par lequel un mouvement de piqûre d'un élément aiguille (3) couplé audit entraînement de piqûre au moyen d'un mécanisme de couplage (28) est entraîné dans le sens de la piqûre, et
- présente un élément de référence de profondeur de piqûre (8) doté d'une surface de référence de contact cutané (11) pour améliorer la reproductibilité de la profondeur de piqûre (S), lequel élément est conçu et disposé de telle façon que la surface de référence de contact cutané (11) est en contact avec la peau au point d'inversion du mouvement de piqûre et que la valeur prédéfinie de la profondeur de piqûre (S) est déterminée par la distance, dans le sens de la piqûre, qui existe entre la surface de référence de contact cutané (11) et la position de la pointe (3c) de l'élément aiguille (3) au point d'inversion du mouvement de piqûre,
selon lequel
la surface de contact cutané de l'appareil de piqûre (2) est pressée contre la peau lors de l'utilisation de l'appareil de piqûre (2) ;
l'élément aiguille (3), durant une phase d'avancement du mouvement de piqûre, est déplacé sur un trajet de piqûre prédéterminé, dans un sens de piqûre, jusqu'à ce que sa pointe (3c) pénètre dans la peau pour provoquer une blessure, et est de nouveau retiré durant une phase de recul du mouvement de piqûre, après avoir atteint un point d'inversion correspondant à la profondeur de piqûre (S) dans la peau ;
au moment où la pointe (3c) de l'élément aiguille (3), durant la phase d'avancement du mouvement de piqûre, passe à travers la surface de référence de contact cutané (11) et pénètre dans une surface cutanée en contact avec celle-ci, l'élément de référence de profondeur de piqûre (8),
doté de la surface de référence de contact cutané (11), est dans une position stationnaire définie par rapport au point d'inversion du mouvement de piqûre ;
l'élément de référence de profondeur de piqûre (8) est conçu et disposé de telle façon que la peau, lorsqu'elle est pressée contre la surface de référence de contact cutanée (11), est stabilisée en termes de déformation de la peau se produisant lorsque l'on pique la peau ;
l'élément aiguille (3) présente un canal capillaire (3b) à travers lequel un liquide corporel peut être transporté hors de la peau ;
ledit élément aiguille, après le point de conversion du mouvement de piqûre, est ramené en arrière dans le sens contraire du mouvement de piqûre jusqu'à une profondeur de piqûre résiduelle ; puis
durant une phase de collecte pour recueillir le liquide corporel, un déplacement conjoint de l'élément de référence de profondeur de piqûre (8) et de l'élément aiguille (3) a lieu dans le sens contraire de la piqûre, avant que la pointe (3c) de l'élément aiguille (3) ne soit totalement retirée de la peau.

7. Procédé selon la revendication 6, **caractérisé en ce qu'**avant de presser l'appareil de piqûre (2) contre la peau, l'élément de référence de profondeur de piqûre (8), doté de la surface de référence de contact cutané (11), se trouve dans une position de départ définie, également par rapport à la surface de contact boîtier-peau (7).

8. Système de piqûre selon l'une des revendications 1 à 5 ou procédé selon l'une des revendications 6 ou 7, **caractérisé en ce que** l'élément de référence de profondeur de piqûre (8) et l'élément aiguille (3), durant une partie de la phase de recul, sont déplacés de manière synchrone de telle sorte que la position relative entre la pointe (3c) de l'élément aiguille (3) et la surface de référence de contact cutané (11) de l'élément de référence de profondeur de piqûre (8) reste inchangée.

9. Système de piqûre selon l'une des revendications 1 à 5 ou procédé selon l'une des revendications 6 ou 7, **caractérisé en ce que** la phase de recul du mouvement de piqûre comprend deux parties, le mouvement de l'élément aiguille (3) étant plus rapide dans la première partie que dans la seconde partie et pendant le mouvement rapide de la première partie de la phase de recul, la surface de référence de contact cutané (11) demeurant dans la position stationnaire.

10. Système de piqûre selon l'une des revendications 1 à 5 ou procédé selon l'une des revendications 6 ou 7, **caractérisé en ce que** l'élément de référence de profondeur de piqûre (8), avant que la pointe (3c) de l'élément aiguille (3) ne soit totalement retirée de la peau, est déplacé depuis sa position stationnaire relativement au boîtier (4) en sens inverse de la piqûre, mais seulement suffisamment pour que la surface de référence de contact cutané (11) reste en contact avec la peau.
